(19) Europäisches Patentamt | European Patent Office | Office européen des brevets

(11) **EP 4 452 096 B1**

(12) **EUROPEAN PATENT SPECIFICATION**

| | |
|---|---|
| (45) Date of publication and mention of the grant of the patent:<br>**15.04.2026 Bulletin 2026/16** | (51) International Patent Classification (IPC):<br>***A61B 17/12*** (2006.01) ***A61B 34/00*** (2016.01) |
| (21) Application number: **22843731.5** | (52) Cooperative Patent Classification (CPC):<br>**A61B 17/12113; A61B 17/12163; A61B 17/1219;**<br>A61B 34/73; A61B 2017/00004; A61B 2017/0084;<br>A61B 2017/00876; A61B 2017/00929;<br>A61B 2017/00942; A61B 2017/12068;<br>A61B 2017/12086 |
| (22) Date of filing: **22.12.2022** | |
| | (86) International application number:<br>**PCT/EP2022/087403** |
| | (87) International publication number:<br>**WO 2023/118400 (29.06.2023 Gazette 2023/26)** |

(54) **DEVICE AND SYSTEM FOR TREATMENT OF A VESSEL**

VORRICHTUNG UND SYSTEM ZUR BEHANDLUNG EINES GEFÄSSES

DISPOSITIF ET SYSTÈME DE TRAITEMENT D'UN VAISSEAU

| | |
|---|---|
| (84) Designated Contracting States:<br>**AL AT BE BG CH CY CZ DE DK EE ES FI FR GB GR HR HU IE IS IT LI LT LU LV MC ME MK MT NL NO PL PT RO RS SE SI SK SM TR** | (72) Inventor: **POUPONNEAU, Pierre**<br>**75015 Paris (FR)** |
| (30) Priority: **24.12.2021 EP 21315292** | (74) Representative: **Hepp Wenger Ryffel AG**<br>**Friedtalweg 5**<br>**9500 Wil (CH)** |
| (43) Date of publication of application:<br>**30.10.2024 Bulletin 2024/44** | (56) References cited:<br>**WO-A1-00/54832          US-A1- 2002 087 077**<br>**US-A1- 2005 119 687** |
| (73) Proprietor: **Artedrone**<br>**75013 Paris (FR)** | |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The invention relates to a device and to a system for treatment of a vessel

It is known to use thrombogenic elements to fill aneurysms.

For example, US 6,315,709 discloses a device and a method for treating vascular defects by filling defects, including aneurysms, with magnetically controlled devices.

US 6,530,934 discloses embolic devices comprised of a linear sequence of flexibly interconnected miniature beads for filling of aneurysms.

However, the devices known in the prior art have several disadvantages. For example, they are not easily guidable in the human body in order to reach a site to be treated, such as an aneurysm. In addition, known devices have fairly complicated architectures, leading to difficulties in terms of economics and reliability.

The navigation to an aneurysm might be challenging, especially with tortuous arteries often found, in particular, in older patients. As a result, the treatment time and the risk of damaging arteries may increase.

In addition, some aneurysm may exhibit a complex shape. Achieving sufficient occlusion of the aneurysm while avoiding occlusion of the vessel may be challenging.

Document US 2002/087077 A1 discloses a magnetic embolization apparatus for embolizing an aneurysm of a blood vessel. The apparatus includes a coiled element adapted for insertion within an aneurysm of a blood vessel, the coiled element shaped to be retained within the aneurysm, and one or more permanent magnetic segments carried by the coiled element to internally induce a magnetic field from within the aneurysm to control a magnetic field controllable embolic to embolize the aneurysm.

Document WO 00/54832 A1 discloses a magnetic coil to be delivered to the site of a vascular defect in a patient.

Thus, the object of the present invention is to overcome the drawbacks of the prior art, in particular to provide a device for treatment of a patient that is reliable, easily guidable in the human body, and is less prone to inducing tissue damage and generally provides a safe treatment and outcome.

This and other objects are achieved by the device according to the invention.

The present invention is defined by appended claim 1. Specific embodiments are set forth in the dependent claims.

A device in the context of the present disclosure may also be a particle or a composition that provides a function, preferably a controllable function, within a patient's body. However, it is conceivable that the device comprises further characteristics such as electronics, motors, sensors, actors, etc. in order to provide more complex functions.

The device according to the disclosure is adapted for treatment of a vessel in a patient. In particular, cavities such as aneurysms such as neurovascular or aortic aneurysms may be treated. The device comprises a filling element, a magnetic element, a controlling line, and a release mechanism. The filling element is preferably an expansive and/or a thrombogenic element. Preferably, expansive elements are hydrogel-based and/or comprise a foam. The release mechanism is adapted to release the magnetic element and/or the filling element such as that at least the filling element can be separated from the controlling line. Preferably, also the magnetic element is separated from the controlling line upon release. Additionally or alternatively, the filling element may be detachable from the controlling line and/or the magnetic element by the release mechanism. As a result, it may be possible to remove the magnetic element with the controlling line, while the filling element remains at a treatment site. The magnetic element is adapted to be guided by an external actuator. Guiding of the magnetic element enables guiding of at least the filling element along a vessel path. Preferably, also the magnetic element and other parts of the device are guided along the same path.

The filling element is adapted to reduce the volume of a vessel position in which it is placed for treatment, either by taking up volume or by forming a thrombus. Preferably, the filling element is activatable and volume reduction takes place after activation. Optionally, the device may comprise an activation mechanism adapted to activate the filling element.

Preferably, the device is in a deactivated state during navigation and can be activated, for example by a stimulus, at the treatment site. A stimulus in this context could, for example, be electricity and/or heat.

In some embodiments of the device, a protective shell may be removable.

Furthermore, in some aspects of the disclosure, the device may be configured to navigate autonomously. Thus, an operator may avoid repeatedly pushing the delivery device back and forth until reaching to the aneurysm. This may reduce the risk of damaging a tissue wall.

An exact delivery is difficult and requires time and very well-trained physicians. One of the solution introduced in this application provides a solution for a progressive and automatic delivery of the filling elements

The device is positionable by loosening or pulling the controlling line. Additionally or alternatively, the velocity of the device may be controllable by means of the controlling line, in particular to release parts of the device (e.g. the filling element and/or the magnetic element) at a specific velocity.

Guiding may include steering (i.e. changing the direction of motion) and/or changing the velocity of an object, in particular slowing down, stopping, and/or accelerating. Guiding may also including keeping a position and/or a velocity steady. Guiding may further include a rotation of the device.

In a preferred embodiment, the device is thus adapted to be carried by a flow of blood after insertion into a vessel. Magnetic forces may be used to guide the device in the vessel to navigate along bifurcations, while the controlling line may be used to slow down or stop the device when needed (e.g. before bifurcations or at a target location for treatment). In addition, while blood flow may in some cases be sufficient to carry a device to a general location of an aneurysm, magnetic forces may be necessary to move a device into an aneurysm. Once placed at a treatment site, for example an aneurysm, the device may thus be moved into an aneurysm. The filling element can be activated, either by an activation mechanism or passively (e.g. dissolution of a layer in blood and/or lapse of time), such as to expand and/or trigger formation of a thrombus, for example to fill an aneurysm. The release mechanism may be used to detach at least a part of the device from the controlling line. Release is done after the device is placed in the aneurysm. Release may take place, however, at any subsequent point, for example before or after the filling element is activated.

The cable and/or controlling line may include a wire, a monofilament wire, a multifilament wire, a wire made of coils, and/or a ribbon. The diameter of the cable may be between 10 and 500 $\mu$m, preferably between 20 $\mu$m and 70 $\mu$m. The cable may comprise or consist of polymers such as nylon, of metals such as stainless steel or an hybrid material made of a polymer and metal.

The controlling line may have an outer diameter in the range of 10 $\mu$m to 1000 $\mu$m, preferably 50 $\mu$m to 500 $\mu$m, preferably 80 $\mu$m to 500 $\mu$m, particularly preferably 100 to 350 $\mu$m, even more preferably about 200 $\mu$m. These values allow for a high flexibility, for example to adapt to curved areas in vessels while still providing sufficient mechanical strength for typical controlling line materials.

The controlling line may comprise a string. The string may be formed of a monofilament or a multifilament. Additionally or alternatively, the controlling line may have a core-shell structure. For example, a monofilament may represent a core of the controlling line while a multifilament may be arranged radially outside of the monofilament along its longitudinal axis to form a shell. The core may comprise or consist of a different material than the shell and/or provide different functions. For example, the core may comprise a nylon filament while the shell comprises electrical wires to conduct electricity.

For example, the controlling line may comprise or consist of any type of Nylon, in particular multifilament Nylon.

The controlling line may comprise a hollow tube. A hollow tube may allow the passage of a fluid, such as a gas or a liquid, or a wire. The inner wall of the hollow tube may be coated to minimize the friction with another element. For example, a coating of PTFE may be employed.

To increase the stability of the hollow tube, in particular such as to withstand internal pressure due to passage of a fluid, the hollow tube may be reinforced. For example, a wire may be arranged in a hollow tube wall, on an inside surface, and/or on an outside surface.

The controlling line may comprise a wire for conduction of electricity and/or heat to parts of the medical device. The controlling line may also comprise two or more wires connected to different parts of the device. The wires may be embedded in the controlling line. For example, the controlling line may comprise a core of a multifilament string and the shell may comprise one or several wires. It will be understood that the wires may also be passed through the controlling line at any point during treatment, in particular if the controlling line comprises a hollow tube as described above. The wires may be electric wires.

Additionally or alternatively, the controlling line may comprise two or more wires arranged substantially along the longitudinal axis of the controlling line, preferably parallel to one another.

For example, a controlling line may comprise a monofilament. Two wires may extend substantially along the controlling line and may be connected to another part of the device, for example the release mechanism. If a multifilament is used, all filaments may consist of the same material, or filaments made of different materials may be used. In particular, the controlling line may also comprise a structure, such as a braided and/or twisted structure.

It is also conceivable that the controlling line has a first portion with different properties compared to a second portion arranged at a distance from the first portion along the longitudinal axis of the controlling line. For example, the controlling line may have first portion, which is arranged at a more distal position than a second portion, which comprises or consists of a monofilament. The second portion may comprise or consist of multifilament. This may provide both flexibility for the navigation in a tortuous network and a high rigidity for retrieving the controlling line. For example, a controlling line with a total length of 2 m may have a distal part with a length of 0.3 m made of a multifilament while the remaining line is made of monofilament.

The controlling line may comprise or consist of a mix of materials. Additionally, at least two materials may be provided with a gradient, for example along the longitudinal axis of the controlling line. For example, the first portion may comprise a first polymer, while the second portion comprises a second polymer. The first and the second polymer may be connected via a gradient blend of the first and the second polymer.

The device may, in particular, not comprise any other elements that extend away, such as cables. If the medical device comprises exactly one controlling line, and said controlling line is selectively detached from the device, the device is then free-floating in its environment.

Preferably, the controlling line is not able to transmit data or energy. It may be made of non-conductive materials, or not be able to conduct electricity along its longitudinal direction for example because of its structure (such as a sandwich structure comprising an insulator). The controlling line may be made of metal, but a connection to the medical device may be unsuitable to transmit electricity, for example because the connection is made of or coated with an insulating material. As such, additionally or alternatively, the device may be unable to receive data or energy through the controlling line.

Preferably, the medical device further comprises a trigger wire. Particularly preferably, the trigger wire may be associated with and arranged in parallel with respect to the longitudinal direction of the controlling line. For example, it may be arranged inside of the controlling line, in particular inside a hollow tube which is part of the controlling line. Alternatively, it may be arranged next to the controlling line as a separate element, but preferably associated with the controlling line. The trigger wire is adapted to trigger a function of the device.

The trigger wire may, in particular, transmit a mechanical or an electrical signal and may in particular trigger the function of releasing a drug, activating the filling element and/or selectively detaching the medical device from the controlling line.

The trigger wire may have a diameter between 10 $\mu$m to 150 $\mu$m, preferably between 20 $\mu$m to 70 $\mu$m. The trigger wire can have a cylindrical shape or strip shape. The trigger wire can be made of a polymer, for example PET, or a metal, for example nitinol or stainless steel. In some embodiments, the trigger wire can transmit a mechanical force to a portion of the device, in particular the magnetic element or the filling element, in order to retract e.g. a protective coating, for example by breaking as a result of the force induced by the trigger wire and subsequent pull back.

In certain embodiments, the controlling line may comprise at least one, preferably a plurality, of optical fibers from transmission of UV and/or visible light. The optical fibers may have a diameter of 100 $\mu$m or less, preferably less than 30 $\mu$m. Delivery of light can be used to expand an expansive material (e.g. filling element), to degrade an protective coating and/or to trigger the release mechanism.

The device may comprise a configurable line which may in particular be arranged between the controlling line and other parts of the device (e.g. the magnetic element and/or filling element) and connect the two.

In a first preferred embodiment, the configurable line comprises a shape memory alloy and is adapted to be formed in a predefined shape. This allows to device to have a substantially straight shape for navigation and attain a more (quasi-)spherical shape upon delivery.

In a second preferred embodiment, the configurable line comprises a thin and highly flexible material (e.g. a string) which connects two or more magnetic elements and/or filling elements. Such a configurable line may be referred to as flexible line. Change from a substantially straight shape to a more (quasi-)spherical shape upon delivery may be attained by means auxiliary magnetic elements on the configurable line. The magnetic elements used to change the shape of the configurable line may be the magnetic elements used for guiding and/or additional magnetic elements. In addition to the magnetic forces, flow forces due to blood flow may also assist in moving magnetic parts on the configurable line closer together.

The controlling line properties are adapted to allow for navigation of the device along curved paths. In embodiments where a configurable line is present, the configurable line may be more flexible than the controlling line, i.e. require less force to be deformed elastically or plastically. Higher flexibility may be achieved by choice of material and/or dimensions. For example, the controlling line and the configurable line may substantially be formed of monofilaments of the same material, but wherein the controlling line has a diameter of 150 $\mu$m and the configurable line has a diameter of 50 $\mu$m.

The controlling line may comprise or consist of a biocompatible material. The controlling line preferably comprises or substantially consists of a material selected from a group of materials consisting of a metal, in particular copper, stainless steel, cobalt-chromium-nickel alloys, titanium, titanium alloys, platinum, platinum alloys, Nitinol, nickel-titanium ternary alloys, nickel-free alloys; metal composites but also polymers, carbon fibres, graphene, a fabric, silk, protein fibers, and carbon nanotubes.

Particularly suitable polymers are aramide, in particular one of Kevlar and Twaron, polyamides (in particular Nylon, i.e. PA 6 and PA 66), polytetrafluoroethylene, silicone, polyurethanes, polyvinylchloride (PVC), bioresorbable polymers such as polyglycolic acid (PGA), polydioxanone (PDO), polylactic acid (PLA, in particular one of PLLA and PDLA, and/or their corresponding copolymers such as P(LA-GA)), poly-$\varepsilon$-caprolactone and its corresponding co-polymers (for example P(LA-CL)). In addition, collagen and chitosan are natural polymers that are equally suited as controlling line materials.

It will be understood that any of the above-listed polymers may be blended, mixed or used as respective co-polymers.

Preferably, the controlling line can be bent into a curve with a curvature radius of less than 3 mm, preferably 1 mm, even more preferably 700 $\mu$m without substantial material stress. The person skilled in the art will understand that the above curvature radii refer to an otherwise straight controlling line (i.e. theoretical stress of 0 Pa at a curvature of 0, i.e. a radius of infinity). In particular, the controlling line may be made of a material and/or structure such that the minimum breaking stress (i.e. the mechanical stress in the controlling line before plastic deformation and/or material failure occurs) is in the range of 0.5-4 MPa. The person skilled in the art understands that it is possible to carry out the invention with a controlling line haver higher breaking stress (i.e. a more robust controlling line). However, higher values may not be required for the invention to work.

The elastic modulus of the controlling line may be in the range of 0.001 to 200 GPa. Preferably, a controlling line comprising or consisting of a polymer material may have an elastic modulus of 0.001 to 5 GPa. A controlling line comprising or consisting of a metal may have an elastic modulus of 30 to 200 GPa. Of course, materials may be mixed, blended, or used in a combination such as a composite material in order to achieve any elastic modulus. In particular, a composite material of polymers and metals may be used to achieve an elastic modulus anywhere in the range of 0.001 to 200 GPa.

The breaking stress of the controlling line is typically not reached when controlling the medical device. If the controlling line is a NiTi wire, the ultimate tensile strength (UTS) may reach $1300 \pm 200$ MPa, for polymers wire UTS may be between 30 and 900 MPa.

The controlling line may have a Young's modulus of 0.01-50 GPa, preferably 1-20 GPa, particularly preferably 1-3 GPa.

The controlling line may have a bending stiffness of 0.001-1 $N \cdot mm^2$, preferably 0.021-0.45 $N \cdot mm^2$. Particularly preferably, the bending stiffness is between 0.002-0.08 $N \cdot mm^2$ or 0.1-0.3 $N \cdot mm^2$. Alternatively, the bending stiffness of the controlling line may be about 0.050 $N \cdot mm^2$.

The controlling line may have a rupture stress of 0.1-5 GPa, preferably 0.1-1 GPa, particularly preferably 0.3-0.7 GPa.

The controlling line may have a cross-sectional area in a plane perpendicular to a longitudinal axis of 0.001-0.1 mm2, preferably 0.004-0.1 mm2, particularly preferably 0.01-0.05 mm2.

In particular, the controlling line may be adapted, in particular by choice of at least one of material, diameter, cross-section in a plane perpendicular to a longitudinal axis, to have a breaking force of at least 8 N, preferably at least 12 N.

The surface of the controlling line may be adapted to avoid friction with a vessel wall and/or thrombogenic effects. This leads to safer treatments as tearing of vessel walls and/or embolisms may be averted.

Preferably, the controlling line comprises a hydrophilic surface, such as a surface functionalized with PEG (polyethylene glycol) or polyvinylpyrrolidone (PVP) or poly(vinyl alcohol) (PVA) or polytetrafluoroethylene (PTFE) or a combination. Such a surface enables wetting by blood thus enables easier and safer navigation in the blood. In addition, a hydrophilic surface can limit protein adsorption on the controlling line and thus prevent triggering of the immune cascade for instance.

Preferably, the controlling line comprises a surface with anti-thrombogenic properties. For example, it may be coated with a material that does not cause substantial thrombosis. In particular, a surface may comprise at least one of phosphorylcholine, phenox, polyvinylpyrrolidone, and polyacrylamide. Additionally or alternatively, the controlling line may be coated with a drug with an anti-thrombogenic effect.

Preferably, the controlling line has a surface that is coated with a hydrogel. The hydrogel may be a synthetic hydrogel and/or a natural hydrogel. Preferably, a hydrogel selected from a group comprising elastin-like polypeptides (ELP), polyethylene glycol (PEG), 2-Hydroxyethyl methacrylate (HEMA), polyhydroxymethacry-late (PHEMA), polyvinylpyrrolidone, polymethacrylic acid (PMA) (as well as other methacrylate-based and methacrylic acid-based polymers), agarose, hyaluronic acid, methyl cellulose, elastin, and chitosan is used. Both synthetic hydrogels (ELP, PEG, HEMA, polyvinylpyrrolidone, PMA) as well as natural hydrogels (agarose, hyaluronic acid, methyl cellulose, elastin, chitosan) may be chemically crosslinked and/or physically crosslinked. Other materials that at least partially reduce friction between the controlling line and the vessel walls may also be used.

The device may comprise a magnetic part coated with an expansive material. Additionally or alternatively, the device may comprise an expansive bead which after activation at a treatment site may be detached from the magnetic part. The device may comprise several expansive beads which are carried during one navigation step. Additionally or alternatively, the device may comprise a front expansive payload consisting or comprising expansive beads attached together and arranged at a distal end of the device. Once expanded and delivered at a treatment site, the front expansive payload may be detached from the magnetic part.

Expansive materials may be activatable by contact with physiological liquids (hydration, chemical reaction) or by application of a stimulus such as heat or electricity.

Examples of expansive materials are hydrogels, for example PVA, PVP, Polyacrylamide, superabsorbent polymers, for example polyacrylic acid (PAA), or polyurethane foams. Hydrogels of PVA may be porous.

Preferably, hydrogel-based expansive elements are used which can be expanded by a stimulus. For example, hydrogels based on poly(N-isopropylacrylamide) (PNIPAAm), poloxamers and/or poly(organophosphazene) (PPZN) may be used and can be expanded by an increase in temperature. Optionally, any one of these polymers may be loaded with magnetic nanoparticles which may be heated up and as a consequence heat up the polymer (expansion by hyperthermia). Polyelectrolyte hydrogels may be expanded by electricity. Hydrogels containing poly (methacrylic acid-co-acrylamide) may be expanded by a change in pH.

Additionally or alternatively, to increase the volume occupied by the device in an aneurysm, the device may trigger a thrombogenic effect leading to the formation of a thrombus which fills a volume of treated aneurysm.

For example, the device the device may trigger blood coagulation inside a cavity. The device may comprise a thrombogenic element which is detachable from the magnetic element once thrombus formation has been initiated and the thrombogenic element is delivered to a treatment site. The thrombogenic element may remain in a thrombus and contribute to its mechanical properties such as the rigidity or stability.

Preferably, the thrombogenic element is a thrombogenic bead. Alternatively, other shapes are conceivable, for example ovoid, cylindrical. Preferably, the thrombogenic element is at least partially curved.

Thrombogenic elements may comprise a bead (or another shape) coated with a thrombogenic material as disclosed herein, a bead made of a thrombogenic material as disclosed herein (e.g. a latex bead), and/or a bead at least partially made of a thrombogenic agent such as a bead made of a silicone matrix with tantalum elements, a porous thrombogenic bead, an expansive thrombogenic bead.

The release mechanism is used to release at least a part of the device from the controlling line such that it remains at a treatment site (such as an aneurysm). The release mechanism may in particular comprise or consist of an element allowing for a localized rupture within the device. The device may have one or several release mechanisms.

To ensure that the device is not further moved by the surrounding flow after release, the device may comprise a second release system. The second release system may provide temporary attachment independently of the first release system and may be activated once the stability of the filling element has been confirmed. Preferably, the second release system comprises a cable adapted a displacement of the proximal part from the remaining part in the cavity.

The second release system may be attached or attachable to the filling element and the magnetic element such as to connect the two. Additionally or alternatively, the second release system may be attached or attachable to the controlling line and the magnetic element such as to connect the two.

The second release system may comprise a string, an adhesive or any other element disclosed in the context of the first release system.

Any mechanism to detach an element from a string-like such as the controlling line known in the art may be employed to this end. In particular, the selective detachment may be triggered by an electrical stimulus, a rotation of a magnetic part (which may be the magnetic element or another magnetic part), a physical action, and/or a chemical action.

For example, the controlling line may be glued to the device, wherein the adhesive connection dissolves in blood or another liquid. It would also be conceivable to adapt an adhesive such that is only dissolve in blood above or below a certain temperature. In one embodiment, a metallic box may be put around the adhesion area. The box is heatable by applying a current. The heating causes at least a partial, preferably full, degradation of the adhesive.

In particular, the controlling line may be chemically linked to the device, wherein the chemical connection is breakable under the influence of a temperature increase, change in pH, electrical stimulus, or similar. For example, an electric current may be used to increase the corrosion rate of a magnesium alloy, which forms part of the release mechanism and which connects the two parts of the device. Additionally or alternatively, elements of the device may be connected to the release mechanism by means of an adhesive. Additionally or alternatively, the device, in particular the release mechanism, may at least partially be formed by ferrous material. Applying an electrical current and/or an electrical voltage to the controlling line may cause migration of ferrous ions from the anode to the cathode, which causes dissolution of the ferrous part. Additionally or alternatively, the controlling line may have an insulating portion to protect a part of the controlling line and/or a part of the device from corroding and dissolving.

In some embodiments, the application of a current on the magnetic part may lead to biocorrosion of its surface, in particular when it is made of iron. The biocorrosion may lead to a progressive dissolution of the surface. Consequently, the contact surface with the filling element may be removed and then the two elements may be separated.

Mechanical release mechanisms are also conceivable. For example, a mechanical interlock, i.e. a first and a second contour that interact with each other such that they connect two elements, may be used. It is particularly advantageous to use a mechanical interlock mechanism in combination with an adhesive, because the connection is then provided through cohesive forces in the adhesive as opposed to adhesive forces between the adhesive surfaces of the device.

In some embodiments, the controlling line may be adapted to transmit an electrical signal that releases a part of the device from the controlling line. Similarly, this may be done by means of a magnet and/or electromagnet (e.g. hyperthermia). The medical device may also receive a wireless signal, for example through a wireless signal receiver, that selectively triggers detachment of a part of the device from the controlling line. In some embodiments, the release mechanism is adapted to be degraded under irradiation, for example with electromagnetic radiation (e.g. visible light, ultraviolet, X-ray), which may for example by transmitted through optical fibers arranged in or in proximity to the controlling line, and/or ultrasound waves.

Additionally or alternatively, the release mechanism may comprise of a biodegradable material such as PGA. PGA with very low molecular weight may be used which loses its mechanical integrity once hydrolysis starts, thus ensuring sufficiently fast degradation. Degradation of the biodegradable material may be further accelerated by stimulus such as heat. A very low molecular weight may be in lower than 10'000 g/mol, preferably lower than 8000 g/mol.

Preferably, the device moves passively and is carried by a bodily fluid and may be slowed down by the controlling line, i.e. the device may travel at a velocity which is lower than the flow velocity. Additionally, but optionally, the device may comprise means for propulsion, i.e. an additional device or a mechanism that can accelerate, maintain or reduce a velocity in the human body. For example, this may be a propeller or an impeller.

The magnetic element may be single element, for example a particle of one of the above materials. Alternatively, the magnetic element may comprise a plurality and/or an assembly of particles. The magnetic element may be coated with a coating.

The magnetic part may have a spherical, cylindrical, cubic., or ovoid shape (e.g. comparable to rugby ball).

The magnetic part can be made of hard ferromagnetic materials such as FePt alloys, Nd-Fe-B alloys, SrO6Fe2O3 alloy, soft ferromagnetic materials such as iron alloys (stainless steel AISI 420C, Fe coated with a protective shell made of graphite for example), nickel alloys or cobalt alloys or a combination of these magnetic elements, and/or ferrimagnetic materials such as iron oxide ($Fe_3O_4$ or $Fe_2O_3$).

The magnetic part may be coated to prevent direct contact with physiological liquids such as blood. Thus, corrosion or cells interactions may be avoided. A protective coating can be made of: metals such as titanium, gold, silver, metal oxides, polymers such as polyurethanes, in particular polyurethanes with polycarbonate backbone, polymers with siloxane chains to improve the resistance to oxidative attacks, silicone (parylene, PDMS), polytetrafluoroethylene (PTFE), ceramics such as silica-based ceramics and/or zirconium-based ceramics (e.g. zirconium dioxide). Additionally or alternatively, the protective coating may also comprise graphite or other carbon-based materials.

The magnetic element can be made of magnetic particles embedded into a matrix. The magnetic particles may comprise hard ferromagnetic materials, ferromagnetic materials, ferrimagnetic materials, and/or superparamagnetic materials.

The magnetic particles can have any suitable shape, in particular cubes, spheres, and/or ellipses. The particles may have a characteristic size (e.g. a diameter) between 5 nm and 50 $\mu$m, preferably between 30 and 100 nm.

The matrix may comprise or consist of a polymer such as PLGA, PDMS, polyurethane and/or ceramics such as SiO2.

The matrix may comprise or consist of a hydrogel. The hydrogel may be degradable by hyperthermia and release biodegradables magnetic particles as a consequence. For example, $Fe_2O_3$ or $Fe_3O_4$ nanoparticles with a diameter between 5 and 50 nm, preferably 5 to 20 nm may be released. The integrity of the gel is reduced with the increase of the temperature. Other stimuli as disclosed herein may be used.

The filling element may comprise an expansive material. The expansive material may comprise a hydrogel that swells upon exposure to blood and/or other bodily fluids. Additionally or alternatively, the device may comprise a scaffold of a shape memory material.

Expansive materials may be used to fill voids in the body such as an aneurysm. Self-expansion may be triggered by a stimuli selected from hydration, heat, in particular heating to 37°C, ions, in particular ions present in human blood such as $Fe^{2+}$, $Na^+$, $Cl^-$, and/or phosphate ions.

Further possible stimuli for expansion may be chemical stimuli, electricity, irradiation with electromagnetic radiation, in particular light, ultrasonic energy, and/or hyperthermia.

In general, the filling element may comprise or consist of an expansive material.

The filling element may be at least partially attached to, attachable to, or formed by the magnetic element. Preferably, the magnetic element and the filling element are fixedly attached or attachable to one another, such that they cannot be moved with respect to one another. Alternatively, they are attached or attachable via flexible means, for example a cord, a line, or a suture.

In particular, the different elements of the device, e.g. the controlling line, the filling element, and/or the magnetic element, may be assembled by a chemical connection (e.g. a glue), a physical connection (e.g. by welding) or a mechanical connection (such as a screw) and/or a release system as described herein.

As a result, the filling element can be navigated particularly advantageously together with the magnetic element.

The filling element may comprise a thrombogenic element which comprises at least one of thrombogenic shape and a thrombogenic material.

A thrombogenic element shall be understood as any element that causes coagulation of blood. It can, in principle, have any shape and can cause coagulation because of the material it is made of, because of its shape, because of a certain surface treatment, or because of release of a thrombogenic agent. Of course, these are merely non-limiting examples of thrombogenic elements.

Particularly preferably, the thrombogenic agent causes blood to coagulate substantially instantaneously when it comes in contact with blood. This allows for targeted coagulation at a specific location and with controlled coagulation time.

Preferred thrombogenic agents include biological agents such as thrombin, fibrin, and collagen. Examples of thrombogenic materials are polymers such as nylon or polyethylene terephthalate, polyethylene oxide-based polyurethane PEU-PEO, latex, metals such as titanium, indium, tantalum. Additionally or alternatively, the thrombogenic element may include electrical contacts to induce electrothrombosis.

The thrombogenic element may comprise fibers, which may additionally be coated with a thrombogenic agent, in particular one of thrombin, fibrin, collagen, or latex. Fibers may be dried fibers and/or be configured to remain dry during navigation in the body.

In some embodiments, the thrombogenic element may induce the formation of a thrombus with a tuned composition. For example, a coating comprising a high concentration of thrombin may lead to the formation of large clots with a high fibrin content, leading to more suitable mechanical properties. For example, these mechanical properties may improve the resistance of the thrombus to compaction induced by the blood flow. Preferably, the thrombin concentration in a thrombogenic element, for example a thrombogenic anchoring coating, is selected between 100 NIH U/mL and 1000 NIH U/mL. As a result, the activation of fibrinogen may be suitable to achieve a content of fibrin in the clot of least of 10%, preferably between 50% and 80%.

Additionally or alternatively, there may be different thrombogenic effects in a first and in a second state of the thrombogenic element. For example, in a first state, the thrombogenic effect may be weaker than in a second state.

Thrombogenic shapes are any shapes that cause thrombosis in blood, for example a shape having relatively sharp edges, small radii of curvature, spikes, or other shapes that may cause formation of a blood clot.

The magnetic element may, in particular, be disposed with a thrombogenic shape and configured as a single element that comprises both the magnetic element as well as the thrombogenic element.

The filling element may comprise at least one defined shape, preferably at least a first and a second defined shape. The defined shape is at least partially curved.

Particularly preferably, the defined shape is a bead or other substantially spherical structure. The defined shape may have an aspect ratio comprised in the range of 0.5 to 1.5.

The defined shape preferably has a characteristic length which represents 50% to 200% of a characteristic length of the magnetic element.

The device may further comprise a configurable line. The configurable line may particularly preferably form a connection between the filling element and the magnetic element.

As a result, the device is adapted to change its conformation to optimize the filling of a cavity such as an aneurysm.

Preferably, the configurable line is formed by a portion of the controlling line. In these embodiments, it will be understood that a portion of the controlling line may be released together with the device by the release mechanism. The release mechanism may thus be arranged along the longitudinal axis of the controlling line

For example, the device with a configurable portion of a controlling line may, under a stimulus partially change its conformation. The configurable line, e.g. the configurable portion of the controlling line, may change its shape from a straight line to a curved conformation.

The configurable line allows to arrange the filling elements in an advantageous manner by changing its at least one of its shape and size, and/or a shape and/or size of its cross-section. For example, the shape of the cross-section may change from circular to flat or ribbon-shaped.

The configurable line may comprise a material that undergoes a structural change such as a crystal structure change or an alignment of monomers.

Additionally or alternatively, the configurable line may change its shape from straight to bent, in particular bent at an angle of 2° to 160°, preferably from 20° to 120°, and or change its radius of curvature by at least 1 mm, preferably 2 mm. In certain embodiments, the configurable line comprises a first and a second magnetic part which are attracted to one another and thus induce bending.

The configurable line may be adapted to be formed into a predefined shape. Predefined may be understood as a shape which, in particular after a shape change, remains substantially constant.

The configurable line may have thrombogenic properties, in particular in a second shape, and may be part of a filling element.

As a result, the configurable line is adapted to form a larger aggregated filling body, comprising multiple filling elements, while navigation in a vessel is still possible in a straight configuration.

The filling element may comprise a compressed and expandable material such as a foam. The compressed material can be adapted to expand into an expanded shape. The expanded shape can be sized and shaped to substantially correspond to the characteristic size and shape of the aneurysm. Typically, aneurysms have a characteristic size (e.g. diameter) of 5 mm up to a few centimeters, in particular 7-25 mm.

Preferably, the filling element comprises an expansive element that can be cut before a procedure in order to fit the size and shape of an aneurysm to be treated. The expansive material may still be compressed for delivery.

The foam may thus adapted to substantially completely fill the aneurysms when expanded therein.

The device may comprise a protective coating, in particular as part of an activation mechanism. The protective coating may, particularly preferably, be arranged radially outside of the filling element.

The thickness of the protective coating may be between 20 $\mu$m and 400 $\mu$m, preferably between 90 $\mu$m and 300 $\mu$m.

The protective coating may comprise or consist of a polymer or a mixture of polymers, for example silicone, polyurethane with a polycarbonate backbone and/or polytetrafluoroethylene. The protective coating may be arranged such as to be under a strain on a distal part of the microrobot. Additionally or alternatively, the protective coating may comprise a weakened portion in the distal part, e.g. by portion of protective coating which is thinner than other portions of the protective coating. When the protective coating is removed after breaking a weakened portion, it may be pulled back such as to remove the protective coating from the attachment element.

To break a weakened portion, the protective coating may be moved back in a proximal direction. The movement may be achieved by an electrostrictive material attached to the protective coating. For example, piezoelectric ceramics (e.g. lead zirconate titanate and/or barium titanate) or piezoelectric polymers (polyvinylidene fluoride) may be employed. Additionally or alternatively, a microelectromechanical systems (MEMS) which is activatable by a current may be used.

Particularly preferably, the protective coating is arranged in a sheath-like manner around the filling element and/or the magnetic element.

Preferably, the protective coating may be adapted to dissolve, either spontaneously in aqueous fluids, or upon triggering by a stimuli. The protective coating may be arranged in a manner covering the filling element, in particular the thrombogenic element. Dissolution of the protective coating may bring elements with thrombogenic properties in contact with blood such as to activate the filling element.

The protective coating may be non-bioresorbable or bioresorbable. Preferably, the protective coating is adapted to substantially prevent liquid penetration in order to avoid a premature activation of the expansive material and/or the thrombogenic element.

In one embodiment, a bioresorbable protective coating can be made of a biodegradable polymer loaded with nanoparticles. The polymer exhibits a low degradation temperature (60-40°C). The nanoparticles can be made of gold, $Fe_2O_3$, $Fe_3O_4$ or silver. The protective coating is degraded by heat generated by the nanoparticles under a stimulus such as light or a magnetic field. As a result, the coating may break into smaller biodegradable parts which may be flushed away by the blood flow.

In another embodiment, the protective coating comprises a membrane. The membrane substantially consists of magnesium powder in a matrix of a bioresorbable polymer. By applying an electrical current, for example via the controlling line, the magnesium powder can be degraded. As a result, the membrane breaks because the magnesium powder particles function as a linker in the membrane. The protective coating thus disintegrates and can be flushed away.

Preferably, the filling element is arranged radially on the outside of the magnetic element. This provides for a particularly compact design allowing for advantageous navigation. For example, the filling element may comprise a thrombogenic shape on the surface of the magnetic element. Additionally or alternatively, the filling element may comprise a thrombogenic coating or an expansive coating.

The release mechanism may be arranged in between the controlling line, preferably a first portion of the controlling line, and

- any one of filling element and magnetic element, and preferably a second portion of the controlling line;
- any two of filling element, magnetic element, and preferably a second portion of the controlling line;
- all three of filling element, magnetic element, and preferably a second portion of the controlling line.

As a result, the release mechanism may always detach the controlling line from at least one other part of the device. In a preferred embodiment, at least the filling element is detached from the controlling line by means of the release mechanism. This allows to release parts of the device, e.g. the filling element, once at least said part of the device is placed in aneurysm. The filling element may then fill the aneurysm, while at least the controlling line and optionally other parts of the device are retracted by an operator.

The magnetic element may navigate into a cavity corresponding to a treatment site, may remain there after treatment, or be detached and removed again. In certain embodiments, the magnetic element does not enter the treatment site.

For example, a device comprising a configurable line may be delivered into an aneurysm together with at least one expansive bead and a magnetic part and detached from the controlling line as a whole. In an alternative embodiment, a device comprising a magnetic bead with a thrombogenic coating may be delivered to an aneurysm and detached from the controlling line. The magnetic bead may remain in the aneurysm and become surrounded by a thrombus.

In yet another alternative embodiment, a device comprising a front expansive payload may be adapted to only deliver the expansive material into the cavity, i.e. the filling element is detached from the rest of the device which is subsequently removed (including the magnetic part).

The disclosure further relates to a system for treatment of a vessel in a patient, preferably a cavity such as an aneurysm. In particular, the aneurysm may be a neurovascular or aortic aneurysm. The system comprises a device as disclosed herein. Furthermore, the system comprises at least one controlling line driver, a magnetic actuator, and a controlling unit.

The controlling unit may further be adapted to activate the release mechanism and/or trigger the activation mechanism of the device. The controlling unit may be an integrally formed with the controlling line diver and/or the magnetic actuator. Alternatively, the controlling unit may be a separate unit which is in operable connection with the controlling line diver and the magnetic actuator, e.g. wirelessly or via a cable.

The system may comprise one or several magnetic actuators. In a particular configuration, one magnetic actuator is use to generate a magnetic field covering the cavity to be treated.

The system may generate and control the stimuli used to activate different functions of the device. The stimuli can be generated by the controlling line driver and transmitted to the device via controlling line.

Additionally or alternatively, an additional element may be embedded with the system to generate a stimulus and may be placeable outside of the patient. For example, and ultrasonic probe, preferably setup on a robotic arm, may be automatically applied on the skin of the patient at an appropriate position determined by the controlling unit.

The additional element may also be placeable inside of the patient, preferably in the vascular network. For example, a catheter can be automatically advanced under control of the controlling unit in the vascular network and the stimulus such as light may be delivered. A robotic pushing system may be present to move the catheter.

The system may include a system to measure the flow velocity inside the cavity. The flow in the cavity may be changed due to the deployment of the device. Hence, the monitoring of the flow velocity and flow perfusion in the cavity allows to monitor the progress of treatment and potential movements of the device. For example, the attachment of the filling element may be confirmed by a steady flow around the element. To improve the detection of the filling element with ultrasound, the element may be echo-genic. For example, gas bubbles may be trapped inside of the filling element. Alternatively, the surface of the filling element may be coated with hollow particles filled with a gas.

To activate the release mechanism, the magnetic actuator may be rotated. The rotation leads to a rotation of the magnetic part of the device. The rotation of the magnetic part leads to a twist of the release system. The rotation may thus be performed until breaking of the release system. Under rotation, the magnetic part may also be moved backwards in order to contribute to the breaking of the release system.

The magnetic actuator is adapted to generate a magnetic field at a predetermined location. Preferably, the magnetic field is predetermined. The magnetic field may exert a force on the medical device, in particular the magnetic part of the medical device, such as to steer the medical device in a preferably predetermined direction. The controlling unit is adapted to balance at least three forces applied on the medical device. Preferably, the controlling unit balances the forces in real time. Preferably, the three forces include at least one of a drag force acting on the medical device by a fluid flow, for example blood in a vessel, a force by the controlling line, and a magnetic force by the magnetic actuator. The controlling line further operates the magnetic actuator and/or the controlling line driver.

A magnetic force may be mainly used to adapt the direction of the device in a blood flow. Hence, the device may have an embedded magnetic element to interact with a magnetic field generated outside of the patient.

A drag force induced by the blood flow may include the displacement of the device along the vascular network which is mainly achieved by the blood flow.

A force exerted by the controlling line may be used to regulate the velocity of the distal part of the device, whereby the controlling line is attached to the distal part. A proximal part of the controlling line is connected to a controlling line driver which releases the controlling line at the targeted velocity. The controlling line may be highly flexible to not impair the navigation induced by the blood flow. As a consequence, the controlling line may not push the distal part of the device.

Other forces may be taken into account by the controlling unit, for example a gravity force acting on the device and/or controlling line.

The controlling line can help to the magnetic navigation. The present invention helps to find a good equilibrium on the different forces applied on the medical device: flow force, gravity force, controlling force and the magnetic force or other potential forces acting on the medical device in order to navigate the medical device along the trajectory path. The system, in particular the controlling unit, can automatically compute the forces and the relations between the forces and define the forces generated by the system, especially the controlling line force and the magnetic force, to ensure that the resulting force moves the medical device along a predefined trajectory and in particular at a controlled velocity.

This balancing model allows also to optimize the distribution of forces induced by the magnetic actuator and the controlling line. The balancing of forces may also be beneficial for optimizing system requirements, such as e.g. lower magnetic fields and/or lower controlling line forces.

The controlling line driver may comprise, preferably consist of, any one of or a combination of a pulley, a linear actuator, a reel, an electric motor, a spindle, a gear, a screw and/or a nut, a linear gear track, and a continuous track. The controlling line driver may also comprise two or more of any one of these elements, also in combination with any one, two, or more of any other element.

For example, two pulleys may be connected to two controlling lines attached to a distal part of the device. The synchronization and unsynchronization of the pulleys is used to orientate the distal part and/or to trigger specific functions of the devices.

The controlling line driver may also comprise, additionally or alternatively, a controlling line connector adapted for providing an operable connection between the controlling line driver and the controlling line.

Preferably, the controlling unit may comprise a processor and/or a memory. In a particularly preferred embodiment, the controlling unit is in operable connection with an electric motor and is adapted to control at least one of speed, power, and torque of the electric motor.

The speed may be at least partially predetermined, automatically determined or manually chosen. It is conceivable to use a combination of predetermined, automatically determined, and manually chosen speeds. For example, the controlling unit may calculate a suitable speed profile based on a planned trajectory in a vessel taking into account data about the flow of blood in said vessel and save the speed profile in a memory. Additionally or alternatively, the speed of the controlling line may be adapted during an intervention automatically, for example via feedback loop taking into account a planned trajectory and actual position data, and/or manually by a user. To this end, the system may preferably comprise an interface for a user, for example one or more touch screens, knobs, buttons, levers, adapted for allowing input of speed parameters. It is possible to use the same or additional interfaces for inputting further parameters related to control of position and speed of the medical device.

Preferably, the controlling unit is adapted to calculate a magnetic field at a device position in space and/or a force exerted on a magnetic element by said magnetic field when the magnetic element is positioned at the device position in space. The controlling unit may in particular take into account at least one of a position, an orientation, and/or a power of the magnetic actuator. Additionally or alternatively, the controlling unit may be adapted for receiving data from a sensor at or close to the device position, in particular data related to the magnetic field and/or force at the device position.

Additionally or alternatively, the device may calculate at least one of a position, an orientation, and a power of the magnetic actuator suitable to achieve a magnetic field and/or a magnetic force at the device position. The magnetic field and/or magnetic force may be calculated qualitatively (e.g. only a direction) or quantitatively.

It is conceivable that the controlling unit is adapted to perform a closed feedback loop with respect to magnetic actuator, i.e. to calculate the position, orientation and/or power of the magnetic actuator based on a desired force and/or magnetic field, and to adapt or correct said position, orientation and/or power, for example based on an actual measured field and/or force.

The controlling unit may further be adapted to calculate and/or determine a force acting on the medical device through the controlling line. To this end, the controlling unit may comprise and/or be in operable connection with a force sensor adapted to to measure a force acting on the medical device. The controlling unit may control the controlling line driver such as to release the controlling line while keeping a force acting on the medical device via the controlling line constant. Additionally or alternatively, the controlling unit may be adapted to control the controlling line driver such as to release the controlling line at a constant speed. Yet additionally or alternatively, the controlling unit may be adapted to control the controlling line driver such as to release to controlling line at a speed and/or with a pull-back force that is determined based on a magnetic force acting on the medical device.

Particularly preferably, the controlling unit may control and/or limit, via the controlling line driver, one of the speed and position of the medical device via the controlling line.

The controlling unit may further calculate and/or determine a drag force acting on the medical device due to a surrounding blood flow. The system may comprise a sensor adapted to measure a blood flow velocity at the device position, for example a Doppler ultrasound device. Additionally or alternatively, force data provided by the force sensor may be taken into account. Additionally or alternatively, the system may comprise a memory device containing flow data acquired before or during a treatment in dependence of a position in a vessel.

Thus, the controlling unit is adapted to balance three forces that may be acting on the medical device. Balancing of a number of forces may in particular be understood as adapting the magnitude of at least one force in response and/or based on at least one other force, preferably all other force, of the number of forces.

The controlling unit may be adapted to increase or reduce the magnetic force acting on the medical device by adapting at least one of the position, orientation and/or power of the magnetic actuator depending on the force exerted on the medical device by the blood flow and/or the controlling line. For example, if a force exerted on the medical device by the blood flow is too low for the medical device to move along a longitudinal axis of the vessel, the controlling unit may adapt the operation of the magnetic actuator such as to at least partially exert a force in a direction substantially parallel to the longitudinal axis such as to propel the medical device forward.

Additionally or alternatively, the controlling unit may be adapted to increase or decrease the velocity of the release of the controlling line depending on the drag force exerted on the medical device by the blood flow and/or the magnetic force exerted on the medical device by the magnetic actuator. For example, the controlling unit may determine that the magnetic force that is available may be limited due to space constraints or distance between a tissue and the magnetic actuator and/or the drag force exerted by blood flow strong enough such that the available magnetic force is not sufficient to move the medical device in an intended direction. Thus, the controlling unit may operate the controlling line driver to slow down and/or stop the release of the controlling line such as to slow down and/or stop the medical device. As a consequence, a smaller magnetic force may be sufficient to move the medical device in a desired direction because the medical device has a lower speed, in particular compared to the surrounding blood flow.

The system may comprise orientation means to orient the magnetic field. The orientation means may be in operable connection with the controlling unit. For fields generated by permanent magnets, the orientation may performed by moving the magnetic actuator, in particular by using orientation means having arms, joints, telescopes, wheels, gears, rails, and others and combinations thereof. In particular, the orientations means may comprise a robotic arm with six degrees of freedom for moving a permanent magnet.

In case of a field generated by a non-permanent magnet, the orientation of the magnetic field can be accomplished by changing the current of the electro-magnets and/or by using orientations means as described for a permanent magnet.

In one embodiment according to the invention, the system is additionally or alternatively intended for treating or diagnosing a patient by using an, preferably implantable, medical device having a magnetic part and a shape, size and surface structure defining a movement component of the medical device when it is dragged by a bodily fluid. The system comprises a magnetic actuator, a controlling unit and a controlling line driver. The controlling line may be attached to the controlling line driver. The controlling line driver is or can be brought in operable connection with the controlling unit such that at least one of the position, a movement and a velocity along the axis of a controlling line when attached to the controlling line driver is controllable by the control unit via the controlling line driver. Additionally or alternatively, the controlling unit may be adapted to at least partially control the position and/or the velocity of the medical device, when in operable connection with the controlling unit via the controlling line attached to the controlling line driver, within a vessel via at least one of the position, the velocity and the movement of the controlling line driver.

The controlling unit may further be adapted to control a position of the medical device within the vessel, preferably in a plane perpendicular and/or in a direction parallel to a longitudinal axis of the vessel, via the magnetic actuator. Preferably, the controlling unit is adapted to take into consideration the drag force exerted on the medical device by the blood flow for actuating the magnetic actuator and/or for controlling the movement or position of the controlling line. Additionally or alternatively, the controlling unit may further be adapted to actuate the magnetic actuator based on the velocity of the controlling line or to adapt the velocity of the controlling line based on the magnetic field generated by the magnetic actuator.

Particularly preferably, the controlling unit is adapted to determine three force components exerted on the medical device by blood flow, magnetic forces and the controlling line respectively and to control the controlling line driver and the magnetic actuator such as to balance the three force components in order to achieve an intended movement of the medical device. The controlling unit may be adapted to simulate the progressive filling of the aneurysm with the devices and/or filling elements. The simulations may be based on flow disruption induced by the presence and/or delivery of the filling elements and the thrombogenic reactions, in particular the activation of the coagulation cascade. The controlling unit may be adapted to monitor the progressive filling of the aneurysm during the treatment by image analysis. To this end, images obtained with medical imaging systems known in the art may be used to determine a filling state of the aneurysm with filling elements, which may be compared with a simulated state in order to release filing elements at the appropriate time.

The release of the filling element is triggered by the controlling unit which sends an instruction to the controlling line driver. The controlling line driver has the appropriates features to generate the different stimuli described above. As an example, the controlling line driver may have an internal power supply connected to the electrical wires of the microrobot.

The disclosure is further directed to a method of treating a vessel, preferably an aneurysm, in particular a neurovascular or aortic aneurysm. A magnetically guidable medical device is used for performing the method, preferably a medical device as disclosed herein. The method comprises the steps of

- magnetically guiding the medical device through a vasculature to a target site, preferably an aneurysm,

Preferably activating the filling element in order to expand the filling element and/or trigger a thrombogenic action Releasing at least a part of the medical device, preferably a magnetic element and/or a filling element, at the target site.

Preferably, the steps are conducted in this order.

The controlling unit may be able to simulate the progressive filling of the cavity. In particular, based on the flow variations or/and on the biological reactions induced by the at least one device, the controlling unit may simulate the progressive occlusion and thrombus formation. The simulations allow to determine the appropriate sequence for the device delivery. The sequence provides the spatial device distribution into the cavity and the time prior to release of the filling element.

To confirm that the filling element is appropriately fixed inside the cavity, the magnetic actuator may be moved by 1 mm to 20 mm, preferably between 1 mm to 5 mm, and imaging of the device may be done, for example by fluoroscopy. If no movement of the filling element is detected, a contrast solution may be injected, allowing to monitor the flow in the cavity and around the filling element. If the flow is sufficiently blocked, release by means of the release mechanism may be performed. In some embodiments, the controlling unit may be adapted to process the described information and activate the release.

Preferably, the steps of the method are repeated at least once, preferably twice.

When the steps of the method are repeated, one set of steps as described above may be performed in a certain order., preferably in the order described above. However, it will be understood that the sets of steps may overlap and any step of one set may be in any order relative to any step of a different set.

For example, when the steps are repeated, magnetic guiding may be performed during or after any of the steps of the first repetition of the steps.

The device may further, in any embodiment disclosed herein, comprise an anchoring element which ensures an attachment of the device at a treatment site, in particular inside an aneurysm and/or on a vascular walls.

The anchoring element functionally serves to provide attachment between at least a part of the device, preferably the whole device, with a blood vessel wall and/or a blood clot.

For example, the device may comprise an anchoring coating on an expansive bead.

Additionally or alternatively, the anchoring element may trigger platelet activation and/or a coagulation cascade. For example, biological elements such as collagen, thrombin, Von Wilerbrand factor (vWF), laminin, thrombospondin, vitronectin, fibrinogen or Thromboxane A2 can be used as an anchoring element, in particular as an anchoring coating.

Additionally or alternatively, synthetic adhesive materials may be employed, for example cyanoacrylates. To avoid adhesion with the protective coating, the adhesive anchoring material may be dried. The material can be processed as fibers, for example to deposit a layer of fibers.

The filling element may comprise or consist of a Janus-type bead. A Janus-type filling element may have at least two different surfaces. For example, a Janus-type bead may have on half of its surface an adhesion layer adapted for attachment to the walls of the cavity. The other half of the surface ensures the adhesion with other filling elements.

For example, a Janus bead may have a collagen coating for attachment with a vessel wall, while the other surface may be coated with fibers which are increasing the contact surface with another filling element.

Moreover, specific attachment between two filling beads may be achieved with "click" chemistry and/or by specific topography allowing a connection by means of a click mechanism between the elements and/or surface properties such as surfaces charged with negative and positive charges.

In some embodiments, the device may be imaged or tracked with an imaging modality, such as MRI, CT scanner, echography, X-ray or fluoroscopy, which may be part of the system according to invention. The imaging allows monitoring, preferably live monitoring, of the device during navigation and treatment of a vessel. In particular, the deployment and the final positioning of the device may be tracked and verified.

In particular, the magnetic part, for example made of Nd-Fe-B and with a diameter of 1 mm, may be detected with a C-arm system. Nevertheless, other components of the device may have to be tracked as well, for example the controlling line, the protective coating, and/or the filling element. Thus, the device may further comprise at least one tracking element. The tracking element may comprise or consist of a barium compound, iodine, tantalum, platinum, and/or bismuth.

Preferably, the tracking element is part of the element it is intended to track, i.e. part of the filling element, the magnetic element, the controlling line, protective coating and/or the release mechanism. The tracking element may be arranged in a material by covalent bond or grafting, for example. Additionally or alternatively, the tracking element can comprise or consist strips, rings, and/or powder associated with an element to be tracked.

For example, platinum strips may be arranged on the surface of an element to be tracked. The platinum strips may have a width of 100 $\mu$m and/or a thickness of 50 $\mu$m. Additionally or alternatively, barium powder may be mixed with a polymer, such as polyurethane, and applied on the surface of an element to be tracked. The thickness of such a coating may be in the range of 1 $\mu$m to 70 $\mu$m, preferably between 5 $\mu$m and 15 $\mu$m. Particles of radio-opaque powder may have a diameter in the range of 20 nm to 3 $\mu$m, preferably 50 nm to 100 nm.

The invention is described in further details in the following figures, showing:

Fig. 1a-1i:       schematically a first method of filling an aneurysm with a device according to the invention;

Fig. 2a-2d:      schematically a second method of filling an aneurysm with a device according to the invention;

Figs. 3a-3e:     schematically a third method of treating a vessel site using a device according to the invention;

Figs. 4a-4e:     schematically a fourth method of treating an aneurysm using a device according to the invention;

Figs. 5a-5b:     schematically the working principle of a configurable line;

Figs. 6a-6c:     schematically a method of arranging devices according to the invention in an aneurysm;

Figs. 7a-7d:     schematically a fifth method of treating an aneurysm using a device according to the invention;

Figs. 8a-8e:     schematically the closure of a vessel defect with a device according to the invention;

Figs. 9a-9f:     schematically a manufacturing method for a device according to the invention;

Figs. 10a-10h:   several embodiments of devices according to the invention;

Figs. 11a-11b:   schematically a first protective coating for use with a device;

Figs. 12:        schematically a device with the protective coating of Fig. 11a;

Figs. 13a-13c:   schematically a working principle of a third embodiment of a protective coating;

Fig. 14:         schematically a working principle of a forth embodiment of a protective coating;

Fig. 15:         schematically a working principle of a fifth embodiment of a protective coating;

Fig. 16:         schematically a working principle of a sixth embodiment of a protective coating;

Fig. 17:         schematically a working principle of a seventh embodiment of a protective coating;

Fig. 18:         schematically a working principle of a eighth embodiment of a protective coating;

Fig. 19:         schematically a further embodiment of a device according to the invention;

Figs. 20a-20b:   schematically yet a further embodiment of a device according to the invention;

Fig. 21:         schematically yet a further embodiment of a device according to the invention;

Fig. 22:         schematically a working principle of an embodiment of a filling element;

Figs. 23a-23d:   schematically different embodiments of thrombogenic elements;

Fig. 24:         schematically an alternative embodiment of a device according to the invention;

Figs. 25a-25b:   schematically the working principle of the device of Fig. 24;

Figs. 26a-26b:   schematically a working principle of a further embodiment of a thrombogenic element;

Fig. 27:         schematically a manufacturing process for a device according to the invention;

Fig. 28-28b:     a controlling line driver for a system according to the invention;

Fig. 29:         schematically the working principle of a system according to the invention;

Fig. 30:         schematically a patient being treated with a system according to the invention;

Fig. 31:         schematically the forces acting on a device according to the invention while navigating in a vessel system.

Fig. 32:         schematically a progressive delivery of filling elements.

Fig. 33:         schematically a device according to the invention.

Fig. 34a-34c:    schematically different states of a device during delivery.

Fig. 35a-35d:    schematically different devices according to the invention.

Fig. 36a-36b:    schematically a device according to the invention with an additional release system.

In the following, for clarity, identical reference signs referring to the identical features are only shown once within the same figure number.

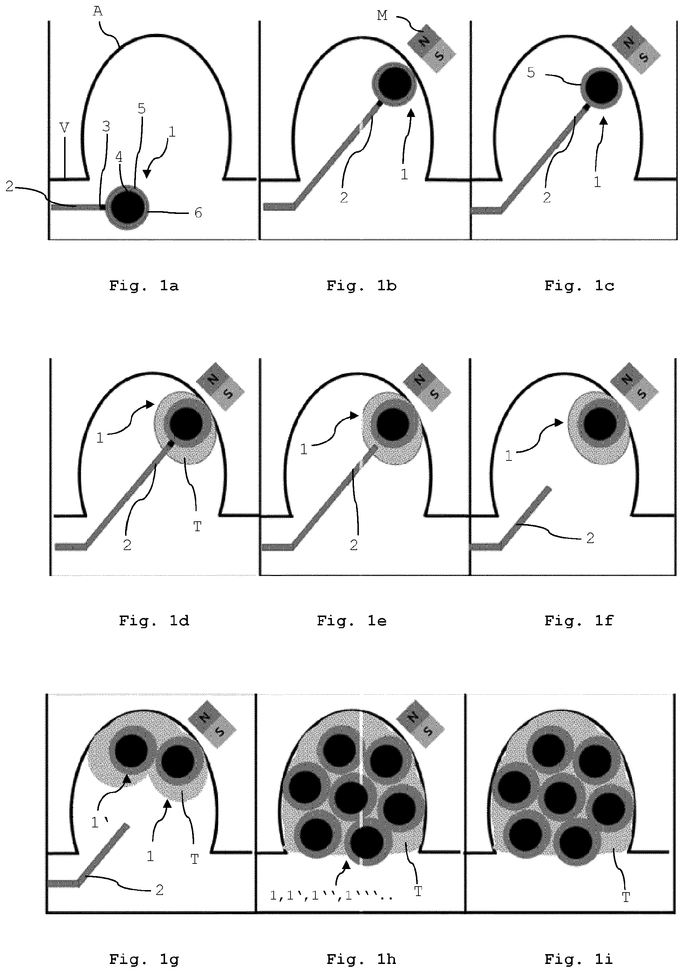

Figs. 1a-1i schematically show the filling of an aneurysm A with a device 1 according to the invention. The device 1 comprises a controlling line 2 made of Nylon with a diameter of 150 $\mu$m to which it is attached via a release mechanism 3 with a diameter of 100 $\mu$m which is degradable by an electrical current. The controlling line is further associated with two electrical wires (not shown) having a diameter of 20 $\mu$m. A magnetic part 4, which has a spherical shape with a diameter of 1 mm and comprising Nd-Fe-B, is provided with a thrombogenic layer made of thrombin having a thickness of 10 $\mu$m and further with a protection coating 6. The protection coating 6 is arranged radially outside of the both the magnetic element 4 and the thrombogenic layer 5, and further the protection coating 6 is arranged in a sheath-like manner around the thrombogenic layer 5. The protection coating comprises PTFE and has a thickness of 10 $\mu$m.

Fig. 1a shows a first step of a method of use of the device 1 according to the invention. The device is navigated up to the aneurysm A in a vascular network V using external magnetic flux provided by a magnetic actuator (not shown).

Fig. 1b shows the device in the general area of the aneurysm A. General area typically refers to a position along a longitudinal axis of a vessel where a cavity is located. A magnetic flux is provided by a magnet M to move the device into the targeted area, here the aneurysm A. The magnetic flux provided by the magnet M may further hold the device 1 in the aneurysm for as long as necessary.

Fig. 1c shows a subsequent step of the method, wherein the device 1 has been moved into the aneurysm A and is located at an appropriate area therein. The protective coating 6 has been dissolved by contact with blood. As a result, the blood is in direct contact with the thrombogenic coating 5 of the device 1.

As a result, a thrombus T starts forming on the surface of the thrombogenic coating 5 of the device 1. The volume of the aneurysm is gradually reduced as the thrombus T. Optionally, a further stimulus may be used to assist the dissolution of the protective coating 6, e.g. an increase of temperature, irradiation with electromagnetic radiation, or others.

Subsequently, as shown in Fig. 1e, the controlling line 2 is detached from the device 1 by means of a release mechanism 3. Here, the release mechanism 3 is degraded by application of an electrical current through the two electrical wires. During navigation, the release mechanism may be protected by the protection coating (see Fig. 1a), in particular if the release system otherwise would be in direct contact with blood.

In a next step, the controlling line 2 is retrieved from the aneurysm A as shown in Fig. 1f (driven by the controlling line driver controlled by the controlling unit, not shown here).

Subsequently, the steps shown in Figs. 1a to 1f may optionally be repeated to deliver a further device 1' into the aneurysm A. The second device 1' may be attracted to the first device 1 by magnetic forces. In particular when hard ferromagnetic materials are used, the magnetic attraction of the first device may be sufficient in some instances to attract and keep a second device in place.

In the shown method, a total of seven devices 1, 1', 1'' are delivered to the aneurysm as shown in Fig. 1h. Magnetic forces lead to an aggregate of the seven devices 1, 1', 1'' inside the aneurysm A. The thrombus T grows to fill the entire aneurysm A. The magnet M may remain in place until complete formation of the thrombus T for additional safety.

Fig. 1i shows the final stage after completion of the treatment, wherein the aneurysm is filled by the device 1, 1', 1'' and the thrombus T. Accordingly, the magnet M has been removed.

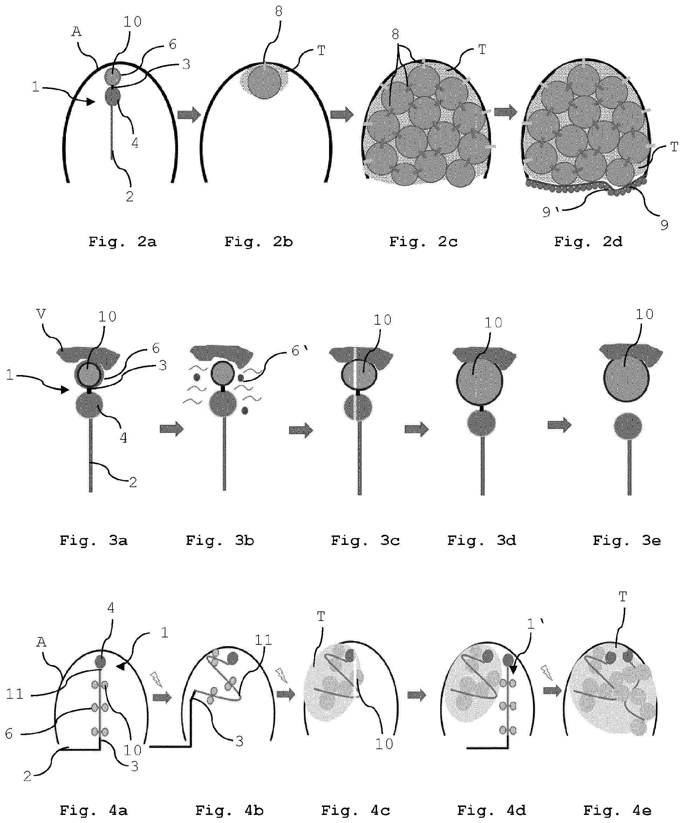

Figs. 2a-2d show a method of treating an aneurysm A using an alternative embodiment of a device 1 according to the invention. The device 1 shown here comprises a filling element formed by a bead 7 of polyacrylic acid with a diameter of 800 $\mu$m and made of a thrombogenic material, which is coated with a protective coating 6. The protective coating is made of polyurethane with a polycarbonate backbone having a thickness of 20 $\mu$m. The bead is attached to the magnetic element 4 via a release mechanism 3. The release mechanism 3 comprises an element made of a magnesium alloy which connects the filling element 7 with the magnetic element 4.

Fig. 2a shows the delivery of the filling element 7 into the aneurysm A. The steps leading up to this stage substantially correspond to the steps shown in Figs. 1a and 1b. Due to corrosion of the magnesium alloy, the release mechanism 3 spontaneously (optionally assisted by electric current) dissolves in the aneurysm and releases the thrombogenic bead 7, which comprises an expansive bead with a collagen coating with a thickness of 20 $\mu$m as a thrombogenic agent. This method avoids that magnetic elements 4 remain inside the aneurysm A after completion of the treatment. It is known in the art that magnesium alloys can have tuneable corrosion rates. As such, the corrosion rate may be adapted such that substantial weaking and dissolution of the release mechanism 3 only takes place after a typical time needed to reach an aneurysm A. In parallel to dissolution of the release mechanism 3, the protective coating 6, which is arranged around the thrombogenic bead 7, is dissolved by contact with blood, leading to activation of the filling element.

Fig. 2b shows the expansive bead 10 after release from the magnetic element 4, dissolution of the protection coating 6 and retrieval of the controlling line and magnetic element 4. Using an anchoring element 8, for example in the form of a thrombin coating, provides adhesion to the aneurysm wall to stabilize the device 1 in the aneurysm. Formation of a thrombus T starts around the expansive bead 10 due to flow stagnation.

As shown in Fig. 2c, the steps shown in Fig. 2a and 2b may be repeated several times to deliver further thrombogenic beads 7 in the aneurysm A. Repetitions may be done until complete occlusion of the aneurysm A is achieved. The expansive beads 7 may be aggregated by means of the anchoring element 8, as cyanoacrylate may also provide adhesion to other expansive beads. The thrombus T which forms eventually fills the entire aneurysm A.

Optionally, after complete occlusion of the aneurysm A, the aneurysm may further be closed off by a membrane 9 with magnetic surface 9'. The magnetic surface 9' is advantageous because the membrane may be oriented and placed using an external actuator (not shown) for optimal closure of the aneurysm. The membrane may adhere to the thrombus T. It will be understood that such a membrane 9 may be used for closure of an aneurysm in combination with any device or system disclosed herein.

Figs. 3a-3e show schematically the working principle of an alternative embodiment of a device 1, which is similar to the device shown in Fig. 2a. The device comprises an expansive bead 10 which is coated with a protective coating 6. The expansive bead is connected to a magnetic element 4 via an anchoring element 3 which substantially corresponds to the embodiment of Fig. 2a. The anchoring element 3 is formed by a collagen layer with a thickness of 10 $\mu$m. Additionally or alternatively, a dried cyanoacrylate adhesive may be used.

Fig. 3a shows the device 1 delivered to a treatment site in a vessel V.

Fig. 3b shows schematically the dissolution of the protective coating 6 comprising gold nanoparticles (not shown) due to irradiation with visible light. The protective coating 6 has a thickness of 20 $\mu$m. The debris 6' resulting from the dissolution of the protective coating 6 is rinsed away by the flow of blood. Light delivery may be achieved through fiber optics forming part of the controlling line and/or a light-delivery catheter. The fiber optics may, optionally, pass through the magnetic element 4.

AS shown in Figs. 3c and 3d, the expansive bead 10 starts swelling due to exposure to blood and reaches larger sizes as time elapses. The diameter may be increased from 0.8 mm to 2.4 mm, i.e. by a factor of three. In parallel, the dissolution of the anchoring element 3 due to corrosion of the magnesium alloy in blood takes place.

Fig. 3e shows a final stages wherein the expansive bead 10 is released from the magnetic element 4 and the controlling line 2 and the expansive bead 10 has reached its final size. Initial adhesion to the tissue wall may occur through natural thrombus formation, but may also be achieved via adhesive compositions or thrombogenic elements as disclosed herein.

Figs. 4a-4e show schematically a method of occluding an aneurysm using a device 1 with a configurable line 11. The device comprises a magnetic head part 4, having a diameter of 1.2 mm and comprising Nd-Fe-B, which is fixedly attached to the configurable line made of Nitinol and having a diameter of 100 $\mu$m. Several expansive beads 10 are attached to the configurable line 11 and coated with a protective coating. Before expansion, the expansive beads 10 have a diameter of 500 $\mu$m. Here, the expansive beads 6 consist of a polyurethane foam which additionally has thrombogenic properties due to a thrombin coating on its surface (not shown). The configurable line in the shown embodiment comprises of a shape memory alloy.

Fig. 4a shows the delivery of the device 1 into the aneurysm. The navigation and movement into the inner volume of the aneurysm may be done substantially as shown in Figs. 1a and 1b. The configurable line 11 has a straight conformation during delivery and when entering the aneurysm 11.

Fig. 4b shows schematically the configurable line 11 changing its conformation from straight to helical spontaneously due to its shape memory effect. It will be understood that other conformations are conceivable as well, for example circular, zigzag, or bent. The release mechanism 3 starts dissolving after the protective coatings 6 arranged on the surface of the expansive beads has dissolved, the latter being expanded as a consequence. Here, the expansive beads 10 were compressed by the protective coating 6.

Fig. 4c shows the device 1 after retrieval of the controlling line 2 subsequent to the complete dissolution of the release mechanism 3. Due to the exposure to blood and the dissolution of the protective coating, the expansive beads expand spontaneously. Due to the thrombogenic properties of the thrombin coating 10, a thrombus T starts forming.

Generally, stagnation of blood due to the occlusion may contribute to thrombus formation. However, such thrombus formation is slow. The use of a thrombogenic agent, for example thrombin, may contribute formation of a thrombus comprising of a fibrin network, leading to advantageous mechanical properties.

Fig. 4d shows the delivery of a second device 1', which is delivered substantially in the same way as described in the context of Figs. 4a-4c.

Fig. 4e shows the complete occlusion of the aneurysm after delivery of both devices 1, 1'' and complete formation of the thrombus.

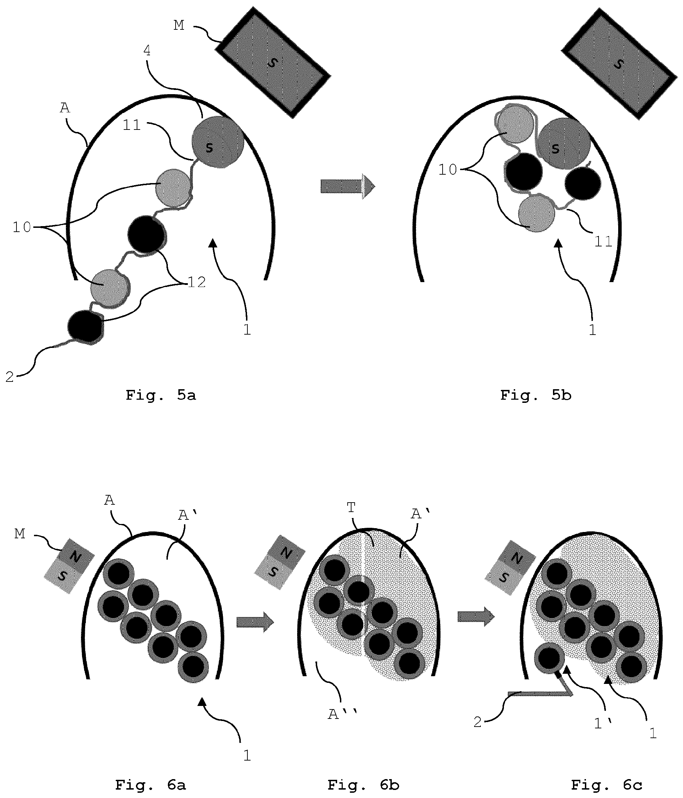

Figs. 5a shows a device 1 with a configurable line 11 in an aneurysm. The configurable line 11 in the shown embodiment is formed of a nylon multifilament string with a thickness of approximately 50 $\mu$m. For clarity, some features of the devices, in particular the controlling line, the release mechanism, have been omitted. The person skilled in the art will understand the navigation of the shown embodiment may have been performed in any way disclosed herein. The device may be combined with any features of other embodiments disclosed, in particular those of Figs. 4a-4e. The device 1 comprises two polymeric beads 10, made of a polyacrylate, with a diameter of 300 $\mu$m which may be thrombogenic and/or expansive. In an alternating fashion along a longitudinal axis of the configurable line 11, two magnetic beads 12 with a diameter of 300 $\mu$m are arranged. The magnetic beads 12 comprise $Fe_3O_4$. The device 1 has a substantially linear configuration which allows for advantageous navigation to the treatment site A under magnetic guiding using an external magnet M. The magnetic beads 12 and polymeric beads 10 are attached to the configurable line 11 by means of a resin.

Fig. 5b shows the device 1 of Fig. 5a after the configurable line 11 has changed its configuration to a substantially elliptical shape. The change in shape in the present example was caused by magnetic attraction of the two magnetic beads 12 to one another as well as the magnetic element 4. As result, the characteristic volume (e.g. defined by the hydrodynamic radius) of the device 1 is increased and better fills the aneurysm.

Figs. 6a-6c show a method of arranging devices 1 in a specific arrangement for advantageous treatment.

Fig. 6a shows a first step of the method, wherein devices are delivered such as to isolate a portion of the aneurysm A' and thus to induce formation of a thrombus (see Fgi. 6b) in this portion. The devices are arranged in a substantially planar aggregate which closes of portion A' of the aneurysm A. The devices are arranged with a preferential orientation, wherein their accumulation causes a stop of blood flow in the portion A' of the aneurysm A. The change in flow velocity in the portion A' area causes thrombus formation.

Fig. 6b shows the formation of the thrombus T, which entirely occludes the portion A'. A second portion A'' of the aneurysm still remains unoccluded.

Fig. 6c thus shows a final step wherein a further device 1' is delivered and released into portion A'' to complete occlusion.

The method shown in Figs. 6a-6c is advantageous because it requires less devices to be delivered compared to full filling of the aneurysm with devices. As a result, the treatment is faster and more economical.

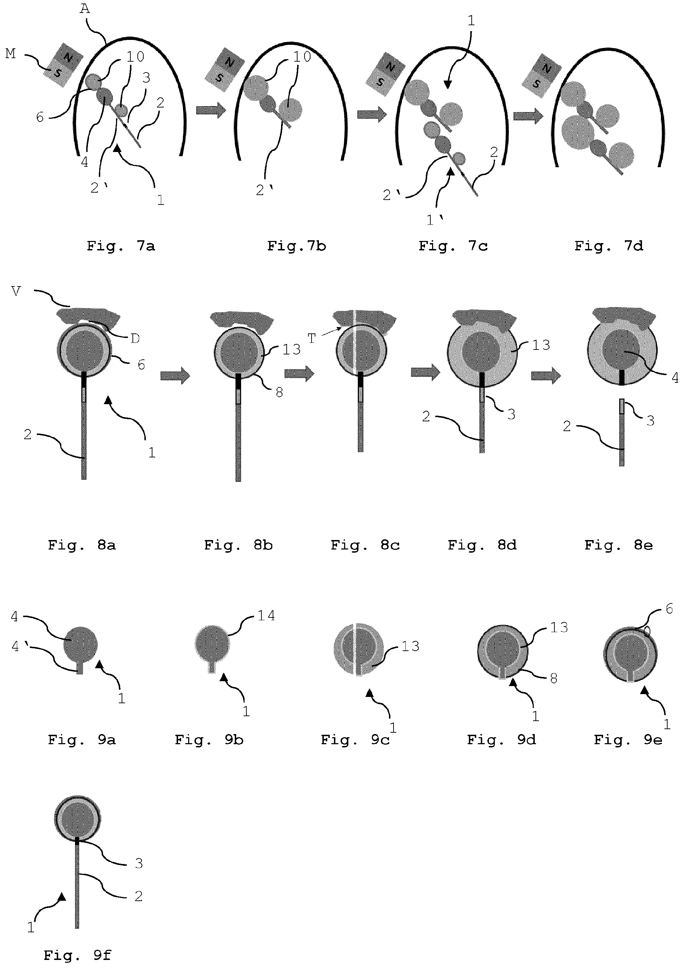

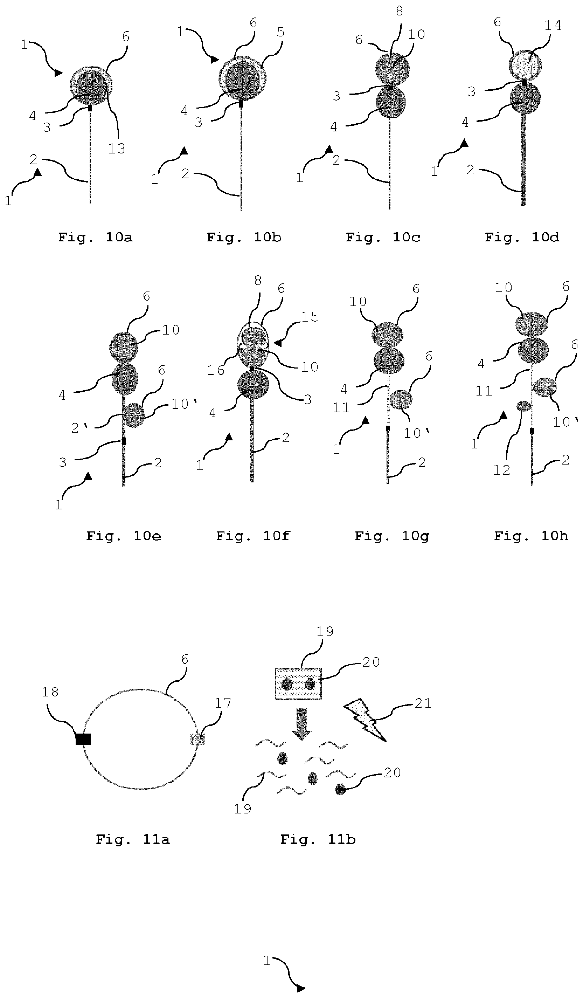

Figs. 7a-7d show an further method to treat an aneurysm A with a devices 1, 1' according to the invention. The device 1 comprises two expansive beads 10 with a protective coating 6, a magnetic element 4, and a release mechanism 3 which connects a first and a second portion 2, 2' of the controlling line. The device 1 will be described in more detail in the context of Fig. 10e.

Fig .7a shows a first step where the device is guided in to the aneurysm A using an external magnet M. While the protective coating 6 is dissolved, the release mechanism is activated to release the second portion of the controlling line 2' for retrieval of the first portion of the controlling line 2.

Fig. 7b shows the device 1 after retrieval of the first portion of the controlling line 2 and expansion of the expansive beads 10 following the dissolution of the protective coating 6.

Subsequently, as shown in Fig. 7c, a second device 1' is delivered to the same aneurysm A.

The second device 1' is expanded substantially as described in the context of Fig. 7a and 7b and released, leading to more complete filling of the aneurysm shown in Fig. 7d.

Figs. 8a-8e show the treatment of a defect D in a vessel wall V using a device 1 according to the invention. The defect may be in an aneurysm.

Fig. 8a shows the device 1 being moved close to a vascular wall V. The device 1 can be held in position by a magnetic field caused by an external magnet (not shown). The protective coating 6 may be removed once the device 1 is in the vicinity of the defect D.

Fig. 8b shows the device 1 after dissolution of the protective coating 6. Directly underneath the protective coating 6 with a thickness of 80 $\mu$m, and arranged radially outside of an expansive coating 13 with a thickness of 800 $\mu$m, the device 1 comprises an anchoring coating 8 comprising collagen. The anchoring coating provides attachment to the vessel wall by formation of thrombus T, as shown in Fig. 8c.

Fig. 8d shows the device 1 after the expansive coating 13, before the controlling line 2 is detached from the rest of the device 2 by means of the release mechanism 3. Expansion of the expansive coating 13 is triggered by exposure to hydration (e.g. from blood) after dissolution of the protective coating 6.

In further step, as shown in Fig. 8e, the release mechanism 3 detaches the controlling line 2 from other parts of the device, namely the magnetic element 4, the expansive coating 13 and the anchoring coating 8, which are left behind to occlude the defect.

Figs. 9a-9e show a method of manufacturing a device 1 according to the invention. The device resulting from the shown manufacturing process is substantially similar to the device shown in the treatment method of Figs. 8a-8e.

In a first step, shown in Fig. 9a, a magnetic part 4 is provided. The magnetic part 4 comprises a stump 4' which can serve as an extension for attachment of the controlling line and/or release mechanism (not shown here).

Fig. 9b shows a next step wherein the magnetic part 4 is coated with an coating 14 with a thickness of 5 to 15 $\mu$m to provide protection against corrosion.

Fig. 9c shows the device assembly 1 after an expansive coating 13 is provided.

In a subsequent step, as shown in Fig. 9d, an anchoring coating 8 is provided on the expansive coating 13.

As shown in Fig. 9e, a protective coating 6 is provided.

Fig. 9f shows the final device 1 after a controlling line 2 has been attached to the other parts of the device shown in Fig. 9e via a release mechanism 3.

Fig. 10a shows a first embodiment of a device 1 according to the invention. The device comprises a controlling line 2, a magnetic part 4, and a release mechanism 3 which connects the controlling line 2 and the magnetic part 4 and allows for their selective separation. Furthermore, an expansive coating 13 is provided on a surface of the magnetic part 4.

Furthermore, a protective coating 6 is provided. It will be understood that the protective coating is an optional feature, but may be particularly advantageous in devices 1 that have an expansive element whose expansion is triggered by contact with blood elements such as water or salts, or devices 1 with an anchoring element (not shown here) embedded on the surface of the expansive coating 13.

The device as shown is typically manufactured by providing a short length of the controlling line 2, which may have a length in the range of 0.2 mm to 20 mm, preferably around 0.5 mm, which is connected to the magnetic part 4. The release mechanism 3 may be attached to the short portion of controlling line 2 in one, preferably the last, manufacturing step. In some embodiments, the release mechanism 3 is positioned such that it lies at the surface of the expansive element 13 after expansion. Hence, the short part of the controlling line 2 used initially together with the magnetic part 4 is embedded in the expansive coating 13.

Subsequently, the magnetic part 4 may be coated with an expansive polymer. The thickness of the expansive coating may be in the range of 10 μm to 300 μm, preferably between 40 μm to 120 μm. A dipping process may be employed to provide the expansive coating 13. A surface treatment of the magnetic part 4 may be done prior to dip coating (or alternative steps of providing an expansive coating 13) in order to improve the adhesion of the expansive material 13 and prevent corrosion. Subsequently, if required, an anchoring coating 8 may be arranged on the surface of the expansive material 13. The expansive material is expandable and shrinkable. The thickness of the anchoring coating may be in the range of 5 μm to 60 μm, preferably between 10 μm and 40 μm. Finally, a protective coating 6 can be provided.

Fig. 10b shows a second embodiment of a device 1 according to the invention. The shown device 1 is similar to the device of Fig. 10a, but comprises a thrombogenic coating 5 instead of an expansive coating. The device 1 further comprises a controlling line 2, a release mechanism adapted to release at least the thrombogenic material 5 from the controlling line 2, a magnetic element 4, and a protective coating 6 preventing premature contact between blood and the thrombogenic layer 5.

The device 1 as shown is typically manufactured in substantially similar way as described in the context of the device of Fig. 10a, wherein the coating with an expansive polymer is replaced with a coating step with a thrombogenic material.

The surface of the magnetic part 4 may be pre-treated in order to improve the adhesion between the magnetic part 4 and the thrombogenic coating 5. For example, the magnetic part surface may be functionalized with carboxylic groups which may covalently bind to the thrombin. The magnetic part 4 may thus be immersed in a thrombin solution with a concentration of 0.4 mg/mL for several hours, typically at least 5 hours. Subsequently, the magnetic part 4 is washed to remove unbound thrombin.

Fig. 10c shows a third embodiment of a device 1 according to the invention. The device 1 comprises a controlling line 2, a magnetic element 4, an expansive bead 10, a release mechanism 3 connecting the expansive bead 10 and the magnetic part 4 and allowing to release one from the other, and a protective coating 6 arranged on the expansive bead. Additionally, the expansive bead 10 may be coated with an anchoring element (8) to provide more secure attachment at a treatment site after detachment from the magnetic part 4.

The device as shown is typically manufactured by attaching a controlling line 2 to a magnetic part 4. The attachment may be achieved, for example, by means of gluing, welding, molding, or melting. Subsequently, the release mechanism 3 is connected with the magnetic part 4. The release mechanism 3 is preferably arranged on a side opposite of the controlling line 2, i.e. on top of the magnetic part 4. The expansive bead 10 is then arranged on the release mechanism 3.

Optionally, the expansive bead 10 may be coated with an anchoring coating 8. The thickness of the anchoring coating 8 may be the range of 10 μm to 150 μm, preferably 20 μm to 50 μm. Alternatively, coating of the expansive bead 10 with an anchoring coating 8 may be done prior the assembly of the expansive bead 10 with the release mechanism 3. This approach is advantageous in that it ensures that the entire surface of the expansive bead 10 is coated with the anchoring coating 8. The expansive bead may be in an expanded state or a shrunk state during coating with an anchoring coating 8.

Subsequently, a protective coating 3 may be coated on the release mechanism 3, the magnetic element 4, and/or the controlling line 2.

Fig. 10d shows a forth embodiment of a device 1 according to the invention. The device comprises a controlling line 2, a magnetic element 4, a release mechanism 3, a thrombogenic bead 14, and a protective coating 6. The device 1 may be manufactured substantially in the same way as described above in the context of Fig. 10c, wherein the expansive bead is replaced with a thrombogenic bead 14.

Fig. 10e shows a fifth embodiment of a device 1 according to the invention. The device 1 comprises a controlling line 2, a magnetic element 4, and two expansive beads 10, 10'. A first expansive bead 10 is connected to the magnetic element 4, and a second expansive bead 10' is attached to a portion of the controlling line 2'. The release mechanism 3 is arranged on the controlling line 2 such as to release both expansive beads 10, 10' together with the magnetic element 4 and the portion of the controlling line 2' to which the second expansive bead 10' is connected. A protective coating is arranged around both expansive beads 10, 10'.

Optionally, at least one of the expansive beads 10, 10' may be coated with an anchoring element (not shown).

It is also conceivable that the first expansive bead 10 may be coated with a different protective coating 6 than the second expansive bead 10' to activate the expansive beads 10, 10' at different times. Such a design may be advantageous when placing further devices 1 in the same cavity.

It is also conceivable that the first and second expansive beads 10, 10' comprise different materials with different expansive properties. For example, one bead 10, 10' may expand more or less or at a different rate than the other. This may be advantageous to fill cavities with complex shapes. A complex shape may be understood as a shape lacking of symmetry.

It may be advantageous if the first expansive bead 10 is arranged below the magnetic element 4, i.e. on the side facing away from an external magnet (not shown). Such an arrangement facilitates the interaction of the magnetic element 4 with another magnetic element or a magnetic field created by an external magnet. In order to be placed and oriented in a particular way, the magnetic element 4 may be arranged with the north and south pole oriented in a specific direction for example along the longitudinal axis of the device or perpendicular thereto.

It will be understood that one or both expansive beads 10, 10' may be replaced with thrombogenic beads as shown in Fig. 10d. This may accelerate occlusion due to the additional formation of a thrombus (not shown).

The device 1 as shown here is typically manufactured by providing a magnetic element 4 a portion of controlling line 2 (see Fig. 10a). The first expansive bead 10 is arranged on the magnetic element 4, preferably on a side opposite the controlling line 2 and/or portion of the controlling line. Optionally, for example if required for the degradation of the protective coating, electric wires may be setup on the surface of one or both expansive beads 10, 10'. Subsequently, the protective coating 6 is arranged on the expansive beads 10, 10'. The release mechanism 3 is then connected to the controlling line 2.

Fig. 10f shows a sixth embodiment of a device 1 according to the invention. The device comprises two expansive beads 10 connected by a linker 16 and coated with an anchoring coating 8, forming a front expansive payload 15. In addition, the device 1 comprises a protective coating 6 around the front expansive payload 15, a controlling line 2, a magnetic element 4, and a release mechanism 3.

It will be understood that one or both of the expansive beads may also be thrombogenic beads. The expansive beads may be arranged linearly and in parallel or orthogonally with respect to a device axis. Three expansive beads may also be arranged in a triangular arrangement.

The linker 16 provides attachment between the expansive beads 10 such as to avoid movement of one bead with respect to another. The linker may comprise an elastic string which is stretched during the expansion of the expansive bead 10. The diameter of the string may be between 30 $\mu$m and 200 $\mu$m, preferably between 50 $\mu$m and 100 $\mu$m. The rope is made of a metal, for example nitinol, or a polymer, such as polyamide. The string may be connected to the expansive beads by gluing. The linker may also comprise a rod which at least partially extends inside the expansive beads 10 is used. The linker may additionally or alternatively comprise an adhesive, for example a glue or a resin, which connects two expansive beads 10.

The front expansive payload 15 may be manufacturing by connecting two expansive beads 10 together with a string acting as a linker. The anchoring coating 8 is subsequently applied on the expansive beads 10 by means of dip coating. After drying of the anchoring coating 8, a first expansive bead is attached to the release mechanism 3. A protective coating 6, for example formed by a polymer film, is placed tightly over the two expansive bead.

Fig. 10g shows a seventh embodiment of a device 1 according to the invention. The device 1 shown here is substantially identical to the device shown in Fig. 10e, except wherein the portion of controlling line 2' is replaced with a configurable line 1.

The configurable line 11 may be bent under the influence of a stimulus. The diameter of the configurable line 11 may be between 30 $\mu$m and 200 $\mu$m, preferably between 50 $\mu$m and 100 $\mu$m. The configurable line 11 comprises or consists of a metal and/or a polymer. For example, the configurable line 11 may be made of nitinol with a diameter of 100 $\mu$m. When an electric voltage is applied, the configurable line 11 experiences increase in temperature and changes its shape, for example to a bent conformation (see Fig. 5b). Alternatively, the configurable line 11 changes into a helical shape due to heating.

The configurable line 11, in particular when made of nitinol, may be connected to electric wires by means of low temperature welding. The electric wires may have a diameter in the range of 30 $\mu$m and 70 $\mu$m. The release mechanism 3 may be arranged such that when the configurable line is released, the welded electrical connections of the configurable line 11 are degraded.

Fig. 10h shows an eighth embodiment of a device 1 according to the invention. The device shown here is substantially identical to the device shown in Fig. 10g, but further comprises a magnetic bead 12 for configuration of the configurable line 11. The magnetic bead 12 is attached to the configurable line 11.

The magnetic bead 12 is adapted to assist in the change of conformation of the configurable line 11 as shown in Figs. 5a and 5b.

The configurable line exhibits a lower bending stiffness than the controlling line, i.e. the Young's modulus of the configurable line may be lower than that of the controlling line 2. Preferably, the Young's modulus of the configurable line 11 may be in the range of 0.5 GPa and 1 GPA, while the Young's modulus of the controlling line may be in the range of 1.5 GPa and 2.5 GPa.

The bending stiffness of the configurable line 11 may additionally or alternatively be reduced by a lower crosssectional area. For example, the diameter of the configurable line may be between 50 $\mu$m and 100 $\mu$m, while the diameter of the controlling line 2 may be between 100 $\mu$m and 250 $\mu$m. The bending stiffness may also be reduced by using a multifilament structure.

In some embodiments, a part of the magnetic bead may be coated with a bioresorbable layer in order to prevent attraction of the magnetic parts during navigation. The bioresorbable layer may increases the distance between the different magnetic elements. The layer thus reduces undesired magnetic attraction. Once the degradation initiated, preferably inside the cavity, the progressive decrease of the thickness ensures a smooth and progressive change in conformation of the configurable line.

The bioresorbable layer preferably comprises or consists of with polymers with fast degradation. Fast degradation may be understood as complete degradation within 1 min to 60 min, preferably 2 to 10 min. Bioresorbable polymers such as PVA, PVP, in particular with low molecular weight, preferably between 5'000 to 40'000 $M_W$ (Da), are used. The biodegradable layer may have a thickness between 10 $\mu$m to 500 $\mu$m, preferably between 50 $\mu$m to 200 $\mu$m. Additionally or alternatively, the biodegradable layer may be coated with a protective coating which is removed at the treatment site.

Figs. 11a shows an embodiment of a protective coating 6. The device is not shown, but it will be understood that the shown protective layer may be combined with any device disclosed herein.

The protective coating 6 is not bioresorbable and typically needs to be removed with the controlling line (not shown). The non-bioresorbable protective coating 6 with a thickness of 10-100 $\mu$m comprises or consists of silicone, polyurethane with polycarbonate backbone, PDMS, and/or PTFE.

To open the coating and expose its interior (i.e. the filling element during a treatment) to physiological liquid, the protective coating comprises a breaking system 17. The breaking system 17 is adapted to be opened or degraded and thus provide an opening in the protective coating 6. An attachment mechanism 18 is connected or connectable to the controlling line 2. Thus, the protective coating 18 may be retrieved together with the controlling line.

The breaking system 17 may comprise a degradable material. The degradable material may be a biocorrodable material (e.g. magnesium or magnesium alloys), a bioresorbable polymer, and/or or a magnetic biodegradable composite. As already described herein, magnesium alloys may corrode faster if a voltage is applied due to electrocorrosion.

Magnetic biodegradable composites may comprise of magnetic nanoparticles such as $Fe_2O_3$ or $Fe_3O4$ and may be embedded into a bioresorbable matrix. The matrix may be degradable by hyperthermia. Magnetic composites are advantageous as they may provide improved attachment between the breaking system and the magnetic element of the device.

The breaking system may also comprise or consist of a bioresorbable polymer as a matrix containing magnesium particles.

In some embodiments, the protection coating 6 is crimped on an element which is at least partially biodegradable. For example, the protection coating 6 may be crimped on a cylinder comprising a bioresorbable part and representing a breaking system 17. Once the bioresorbable part degraded, the blood flow penetrates and hydrates the expansive material.

Fig. 11b shows an embodiment of a biodegradable material which may be used for either protective layers and/or release mechanisms. The material comprises a biodegradable polymer 19 as a matrix loaded with nanoparticles 20. The polymer may have a low degradation temperature of 40-60°C. The nanoparticles comprise or consist of gold, $Fe_2O_3$, $Fe_3O_4$ and/or silver. The biodegradable polymer may thus be degraded by heat generated by the nanoparticles under a stimulus such as light and/or a magnetic field 21.

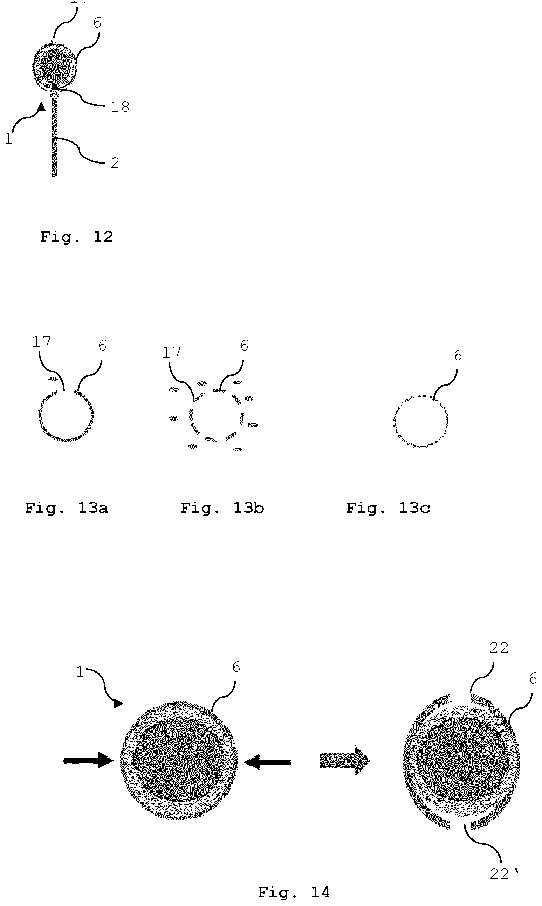

Figs. 12a shows an embodiment of a device with a non-biodegradable protective layer 6 substantially similar to the embodiment of Fig. 11a. The protective layer is crimped on a degradable element 18. A ring 18, to which the protective layer 6 is also crimped, provides attachment to the controlling line 2.

It is also conceivable that the protective layer comprises an area with low mechanical strength as a breaking element 16. A mechanical stress may be applied to the area, leading to tear and blood flow penetration.

Reduced mechanical strength can be achieved by reducing the thickness of the material. For example, the thickness of the protective layer may be around 200 $\mu$m, while the breaking element may have a reduced thickness of approximately 70 $\mu$m.

Additionally or alternatively, the material of the protective layer may be pre-stretched to induce a stress in the material such as to form a breaking element.

Breaking of the breaking element, by exerting a force on the protective layer, may be achieved by expansion of the expansive material. For example, an expansive bead made of foam may be activated by injecting a saline solution through the controlling line.

The breaking element may also comprise a metallic element that can be heated up such as to break the protective coating locally.

Figs. 13a shows schematically a protective coating 6 with exactly one breaking system 17 which provides for local breaking.

In an alternative embodiment, as shown in Fig. 13b, multiple breaking system 17 are present and provide for breaking of the protective coating 6 into multiple parts.

Fig. 13c shows a degradable protection coating which provides for slow and progressive penetration of blood, at a substantially uniform rate over the entire surface.

Fig. 14 shows an embodiment of a device 1 with a protective coating 6 which breaks under a mechanical pressure. The protective coating is adapted to break at two breaking points 22, 22'. The mechanical pressure may be achieved by two plates (made of metals like stainless steel or polymers like PTFE) activated by two rods connected to the release system. The plates have a thickness of 50 $\mu$m with length and width of 100x50 $\mu$m. The rod is a metallic rod (Nitinol) with a diameter of 50 $\mu$m and activated by a current.

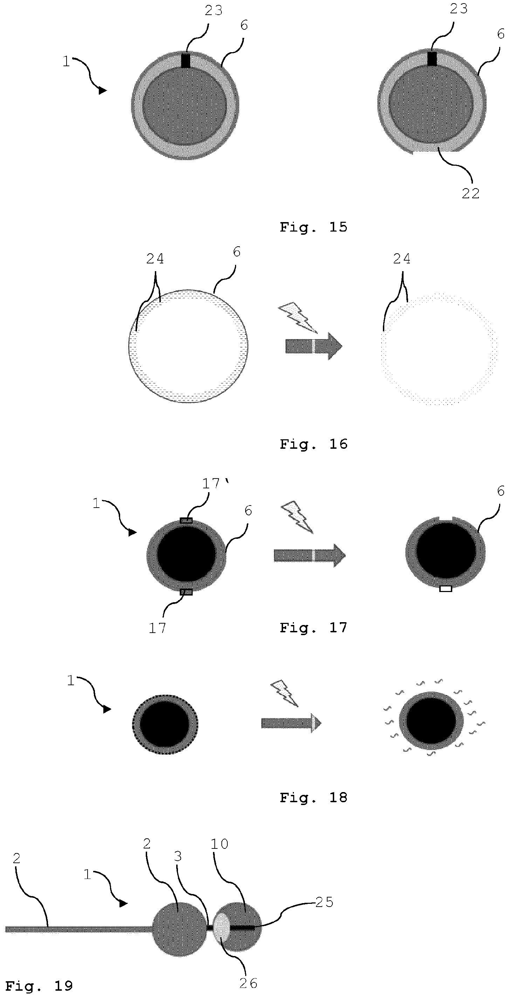

Fig. 15 shows a further embodiment of protective coating 6 that breaks at a breaking point 22 due to local shrinkage in an area 23 opposite the breaking point 22.

Fig. 16 shows another embodiment of a protective coating 6 which breaks due to the presence of local defects 24 in the protective coating material, which may be triggered by any stimulus disclosed herein and/or mechanical stress.

Fig. 17 shows an embodiment of a protective coating 6 with two breaking systems 17, 17' which may degrade spontaneously or under the influence of light, electricity, ionic solutions and/or mechanical stress. One of the breaking systems 17, 17' may also form the release mechanism and detach the device 1 from the controlling line. The remaining parts of the protective coating 6 comprising a bioresorbable polymer may be flushed away by blood.

Fig. 18 shows an embodiment of a protective coating 6 comprising an activatable polymer with a thickness of 10 to 50 $\mu$m and that desintegrates under the influence of light, electricity, ionic solutions and/or mechanical stress. The protective coating 6 thus breaks apart into fragments that may be flushed away by blood.

Fig. 19 shows a further embodiment of a device 1 according to the invention. The device is similar to the device shown in Fig. 10g. The device 1 additionally comprises a rod 25 and a foam neck 26. The rod 25 may connect two different expansive elements. For example, two different expansive materials may be integrated in one expansive bead 10 and connected by the rod 25, in order to provide for different expansion rates. The rod may comprise or consist of stainless steel and have a diameter of 100 $\mu$m and a length of 700 $\mu$m. Additionally or alternatively, the shape of a first expansive element may be substantially spherical, while a second expansive and the second expansive element has a substantially ellipsoidal shape.

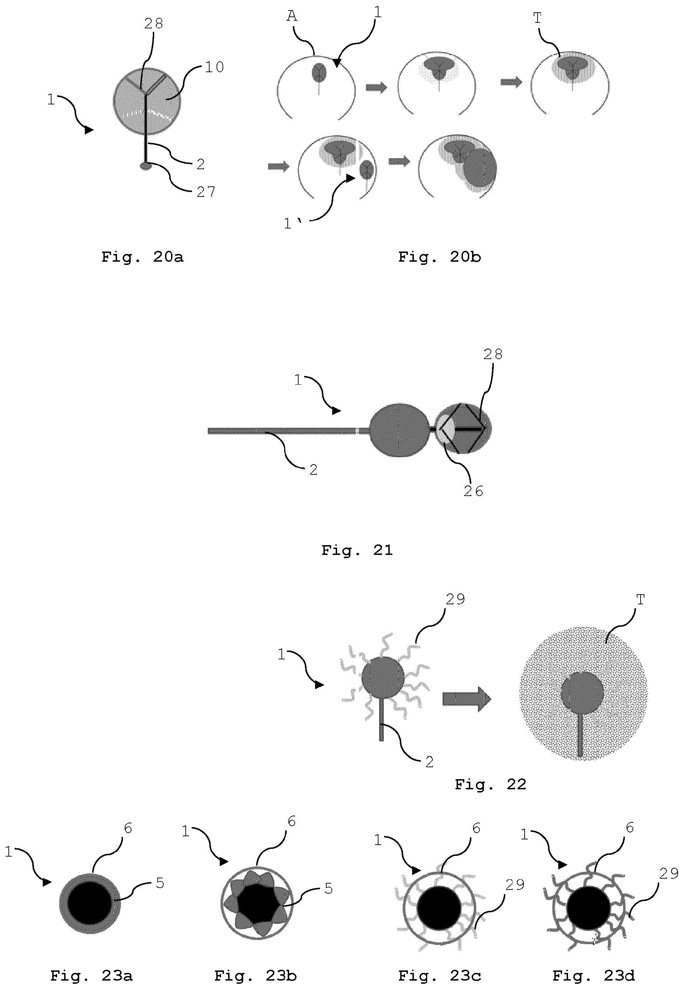

Figs. 20a shows a device 1 with a frame 28 arranged within an expandable bead 10. The frame 28 can be heated when electrical current passes through, leading to the expansion of the expandable bead 10. The device 1 further comprises a releasable bullet 27. Additionally or alternatively, the frame 28 provides increased rigidity of the expansive material, i.e. the expansive bead 10, to withstand compression. For example, compression may be induced by a physiological fluid. The frame 28 may comprise or consist of nitinol. The thickness of one arm may be between 20 $\mu$m and 500 $\mu$m, preferably between 70 $\mu$m and 150 $\mu$m Fig. 20b shows the device of Fig. 20a while treating an aneurysm and forming a thrombus during progressive deployment.

Fig. 21 shows an expansive material integrated into a frame with a complex shape, for example an octahedron. Preferably, an expansive material is coated on the arms of the frame 28. The thickness of one arm may be between 20 $\mu$m and 500 $\mu$m, preferably between 70 $\mu$m and 150 $\mu$m.

In some embodiments, the expansive material may be integrated into a crimped frame. A crimped frame may have a stent-like shape and be expandable. The crimped frame may be coated with an expansive material, for example with a layer or as fibers. The crimped frame may also be employed to break the protective coating by providing an inner force and tension on the protective coating, as described above.

It is also conceivable that the frame comprises individually activatable and selectively parts. This design allows to open the frame in a specific, user-defined shape which may be adapted to the geometry of the cavity to be occluded. For example, each crimped frame part may be connected to individual electric wires. Hence, a frame elements are selectively expanded when a current is applied to the corresponding element.

Fig. 22 shows the basic principle of a device 1 with thrombogenic fibers 29 as filling element. The fibers may either be made of thrombogenic material or a polymer be coated with a thrombegenic material, in particular collagen or latex. The fibers may have a diameter of 0.1 to 500 $\mu$m, preferably 10 to 80 $\mu$m. Here, the fibers comprise PLGA with a diameter of 20 $\mu$m and which are coated with collagen. Suitable other coating materials are thrombin and fibrin. The fibers may be dried. Once exposed to blood, the thrombogenic fibers cause formation of a thrombus T around the device **1.**

Fig. 23a-23d shows different embodiments of thrombogenic elements on a device. All the devices 1 shown here comprise a protective coating 6 which may be any protective coating disclosed herein. It will be understood that the conceptual depiction of the devices 1 here may be complemented with any compatible features disclosed herein.

Fig. 23a shows a device with a thrombogenic material 5 grafted on the surface of the device. The thrombogenic material 5 may be an enzyme, a protein, a polymer, and/or metal.

In general, any substance or material which may trigger blood coagulation and/or platelet activation may be a suitable thrombogenic material.

For example, biological elements such as collagen, thrombin, Von Wilerbrand factor (vWF), laminin, thrombospondin, vitronectin, fibrinogen or Thromboxane A2 may be used. Thrombin is an enzyme which turns fibrinogen into fibrin via cleavage of fib-rinopeptide **A.** A formed fibrin network can capture erythrocytes and platelets leading to formation of a thrombus.

Polymers may also have a thrombogenic effect. Suitable polymers are PET, PEU-PEO and/or latex. Coatings of such polymers may be made by dip coating, spraying, and/or chemical vapor deposition.

Metals which induce a thrombogenic effect may include titanium, indium, and/or tantalum. Metals may be deposited by CVD, plasma spraying and/or physical vapor deposition.

Fig. 23b shows an embodiment of a device 1 with a thrombogenic element 5 whose thrombogenic effect is due to the shape of the material. As such, the thrombogenic element may be made of any suitable material. It will be understood that a thrombogenic material may be used in addition for increased thrombogeneicity.

The shape is adapted to trigger red blood cells hemolysis and thus platelet activation leading to the formation of a thrombus. For example, grooves with a height between 100 $\mu$m and 300 $\mu$m. Preferably, an hydrophobic surfaces are used to increase protein and platelet adhesion.

Fig. 23c shows an embodiment of a device 1 with thrombogenic fibers 29 which are made of a thrombogenic material. The fibers are deployable upon hydration. The fibers 29 may be attached to the device by grafting, spraying, and/or electrospinning.

Fig. 23c shows an embodiment of a device 1 with thrombogenic fibers 29 which are further coated with a thrombogenic element, in the present example thrombin. Such a design is particularly advantageous in that it contributes to holding a formed thrombus around the device 1. Fibers generally provide an enhanced thrombogenic effect. The fiber diameter may be in the range of 50 nm to 50 $\mu$m, preferably between 800 nm and 3 $\mu$m.

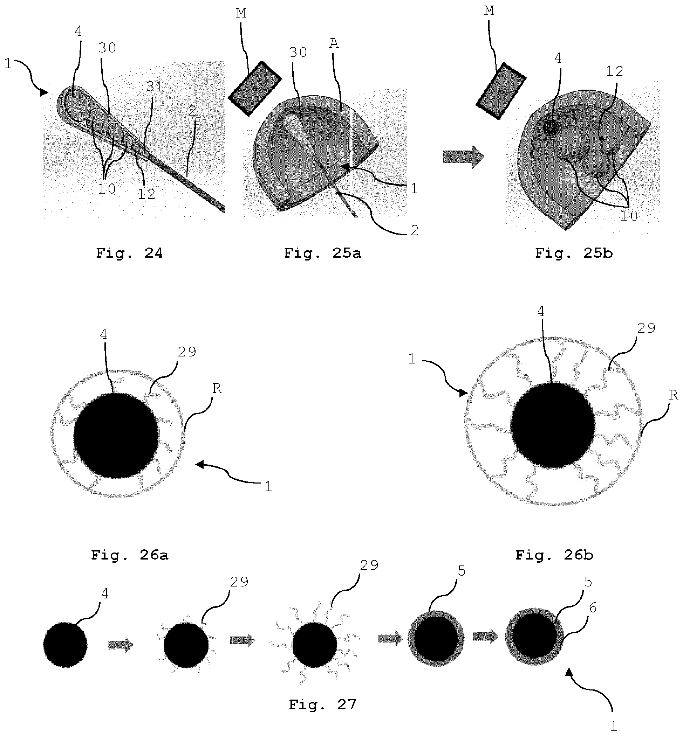

Fig. 24 shows an embodiment of a device 1 according to the invention. The device 1 comprises a controlling line 2, to which is attached a flexible shell 30, which is closed by a plug 31. The controlling line 2 comprises multifilament nylon (diameter of 200 $\mu$m) with two electric wires (diameter 20 $\mu$m) connected to the plug 31. The flexible shell has a thickness of 20 $\mu$m and comprises polyurethane. The flexible shell comprises a breaking point with a reduced thickness of 10 $\mu$m. The plug 31 is made of a magnesium alloy and is degradable by corrosion upon application of a current. A magnetic bead 12, comprising Nd-Fe-B and having a diameter of 1 mm, to adapt the conformation of a configurable line (not shown), to which the expansive beads may be attached, is contained in the flexible shell 30. Inside the flexible shell 30, the device 1 comprises a magnetic element for guiding/navigation in a vascular system. In addition, three expansive beads 10, having diameters of 400 $\mu$m, 500 $\mu$m and 600 $\mu$m, respectively, are arranged inside the flexible shell. After degradation of the plug 31, hydration by exposure to blood may trigger expansion of the expansive beads 10. Optionally, an additional magnetic part with a diameter of 300 $\mu$m, for example made of $Fe_3O_4$, may be used to orient the expansive beads inside the aneurysm. The flexible shell may be made by melting.

Fig. 25a shows the device 1 of Fig. 24 after navigation into an aneurysm under magnetic guiding by an external magnet M. Before retrieval of the controlling line 2, the plug 31 is removed. As a result, the expansive beads are hydrated by exposure to blood.

Fig. 25b shows the three expansive beads which are connected via a configurable line (not visible). At each ends of the configurable line, there is a magnetic part formed by the magnetic bead 12 and the magnetic element 4 ensuring a compact overall shape. It will be understood that the order shown here (magnetic element 4, expansive beads 10, magnetic bead 12) is of exemplary nature and may be changed to any other order without departing from the inventive thought.

Fig. 26a shows an embodiment with self-expanding fibers 29. The fibers 29 may comprise or consist of self-expanding material, for example a superabsorbent polymer. Additionally or alternatively, the fibers may have a core-shell structure. The thrombogenic material is preferably arranged in the shell.

Fig. 26b shows the device of Fig. 26a after the fibers have self-expanded. As a result, the hydrodynamic radius R of the device 1 is increased.

Fig. 27 schematically shows a manufacturing method for a device 1 according to the invention. A magnetic element 4 is provided and swellable thrombogenic fibers 29 are attached to its surface. The fibers 29 are a allowed to swell, for example in saline, and subsequently dried, leading to a thrombogenic layer 5. A protective coating 6 is provided on the thrombogenic layer 5.

A controlling line may be added to the device at any point.

Some embodiments may be adapted to increase the blood temperature around the device. For example, a metallic piece is put on a distal part of the magnetic element. The metallic part may be heated by applying a current. Additionally or alternatively, the metal piece may be heated by hyperthermia and/or due to exposure to electromagnetic radiation or ultrasound. For hyperthermia, the element may be made of superparamagnetic nanoparticles.

These devices are particularly advantageous as the protective coating may be dispensed with, as the thrombogenic effect is only induced the temperature is increased.

It will be understood that in general, any expansive element shown herein may be replaced with a thrombogenic element, and vice versa.

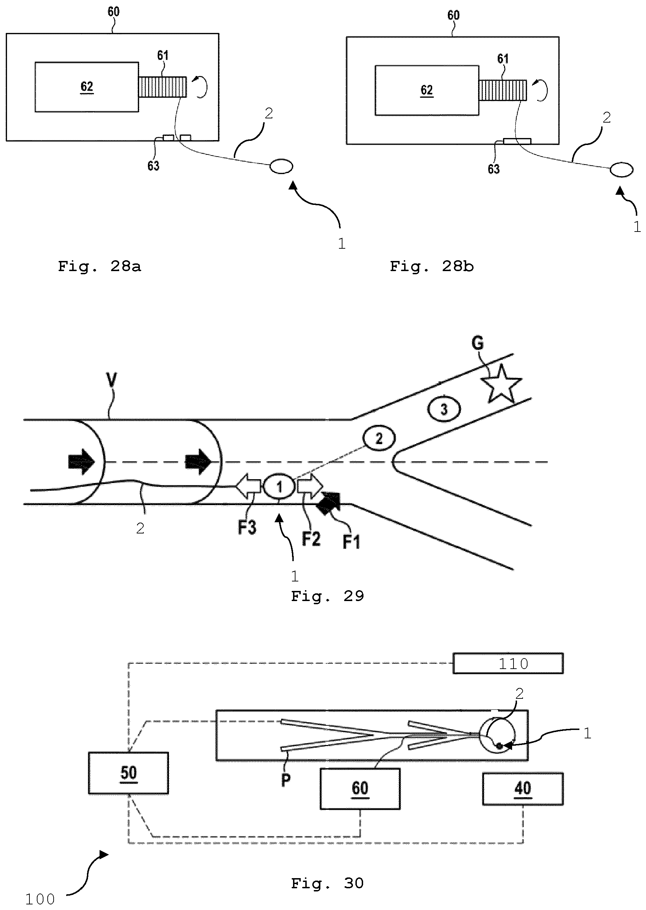

Fig. 28a shows an embodiment of a controlling line driver 60. The controlling line driver 60 comprises and electric motor 62 For clarity, a connection to a controlling unit (see Fig. 30) is omitted. It will be understood that the controlling unit may be connected to the controlling line driver 60 by any means known in the art, such as cables or wireless connections (such as Bluetooth, wireless LAN, infrared ports, or others). The controlling line driver 60 further comprises an axle 61 which is in operable connection with the electric motor 62. The axle is adapted to receive a controlling line 2, which can be rolled around the axle 61. Rotation of the axle 61 releases or pulls in the controlling line 2. The controlling line driver 60 further comprises a clamp 63. Here, the clamp 63 is shown in an open arrangement, allowing the controlling line 2 to move freely through the clamp. Thus, release, stop, or pull-in of the controlling line 2 is controlled only via the axle 61. The position of the medical device is controllable by the controlling line driver 60 and the axle 61 via movement of the controlling line 2.

Fig. 28b shows the controlling line driver 60 of Fig. 1a, wherein the clamp 63 is shown in closed configuration. Thus, the controlling line 2 can be stopped and held independently of movement of the axle 61. For example, the medical device 1 may temporarily be held and secured without using power for the electric motor 62. Closing the clamp 63 to hold the controlling line 2 may also be advantageous to secure the medical device 1 via the controlling line in case of a malfunction of the controlling line driver 60. The clamp 64 may, however, be used to stop and/or hold the controlling line 2 at any time and for any reason.

Fig. 29 shows a medical device 1 which is navigated by a system according to the invention. The medical device 1 comprises a controlling line 2 which provides a third force F3 to the medical device, in addition the blood drag force F2 and the magnetic force F1. Because the system detects the drag force F2, the system may, via the controlling unit (see Fig. 9) balance and set the pull-back force F3 and the magnetic force F1 such that the total force acting on the medical device 1 moves the medical device to a second position and subsequently to a third position in the direction of a target area G.

Fig. 30 shows a system 100 according to the invention. In addition, the system 100 shown here further comprises an imaging system 110. The imaging system may in particular be an ultrasound imaging system, a Doppler ultrasound imaging system, a fluoroscope, PET scanner, CT scanner, MRI system, or any other imaging system known in the art.

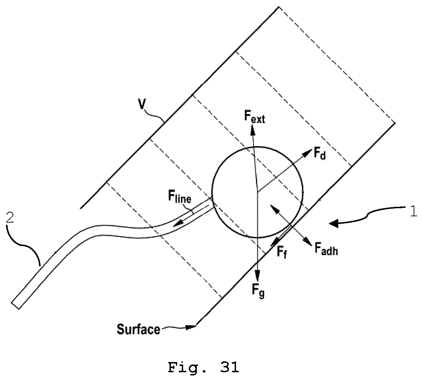

Fig. 31 schematically illustrates a force balancing model which may be used to with a device and system according to the invention. A medical device 1 is shown in a vessel including a controlling line 2. The forces acting on the medical device 1 are:

- Hydrodynamic force $F_d$
- Gravitational force $F_g$
- Adhesion force $F_{adh}$
- Friction force $F_f$
- Normal contact force on the surface $F_n$
- Magnetic force $F_{ext}$
- Force $F_{line}$ exerted by the controlling line 2

The hydrodynamic force $F_d$, the gravitational force $F_g$, and the adhesion force $F_{adh}$, Friction force $F_f$ and the normal contact force on the surface $F_n$ are forces that occur inherently when a medical device 1 is immersed in a blood stream. The magnetic force $F_{ext}$ and the force $F_{line}$ exerted by the controlling line 2 are artificially exerted and controlled by the system according to the invention.

The combination of the different forces described above determines the speed vector (orientation & intensity) and therefore the movement of the medical device 1 in the vessel V.

The motion of the medical device 1 may be described using the following equation (where m is the mass of the medical device and $v_r$ its velocity) :

$$m\frac{dv_r}{dt} = F_g + F_d + F_n + F_{line} + F_f + F_{adh} + F_{ext}$$

The purpose of the force balancing is to determine necessary forces to be exerted by the system that, combined with the naturally occuring forces will generate a speed vector suitable to move the medical device 1 along a pre-determined trajectory.

In particular, the force balancing as described above allows to stop, to pull back, to take a bifurcation and/or to steer the medical device 1 in a vessel with reduced flow.

The different forces can be pre-determined, estimated, measured directly, measured indirectly, or neglected.

To illustrate the above, two exemplary calculations using the above model are shown. Two sizes of medical devices (1.2 mm and 0.6 mm) are modelled for two different bifurcations (internal carotid artery segment 1, hereinforth

23

ICA1-R, to internal carotid artery segment 2, hereinforth ICA2; and ICA2 to anterior cerebral artery segment 1, hereinforth ACA1):

A medical device 1 with a size of 1.2 mm would need to be subjected to a magnetic force of $2.9 \cdot 10^{-6}$ N by a magnetic actuator having a volume of 400 cm$^3$ and which is placed at a distance of 45 cm to pass the ICA1-R to ICA2 bifurcation. To pass the ICA2 to ACA1 bifurcation, a force of $8.9 \cdot 10^{-5}$ N and a distance of 17 cm is necessary.

Similar calculations for a medical device 1 with a size of 0.6 mm yield $3.7 \cdot 10^{-6}$ N at a distance of 24 cm (ICA1-R to ICA2) and $5.4 \cdot 10^{-5}$ N at a distance of 10 cm (ICA2 to ACA1).

The equation shown above allows to estimate the acceleration (i.e. speed variation) of a medical device taking into the different forces acting on it.

Typically, all force acting on the device 1 are known except the force $F_{line}$ of the controlling line 2. Thus, the force balance equation may be solved by either one of two ways:

- estimate the force exerted by the controlling line 2 and compute the acceleration of the medical device 1
- determine the acceleration of the medical device and compute the resulting force exerted by the controlling line 2.

There is an equivalence between the acceleration of the medical device 1 and the force exerted by the controlling line force 2. It is possible to determine force exerted by the controlling line 2 from the acceleration of the medical device 1 or to determine the acceleration of the medical device 1 from the force exerted by the controlling line 2.

For example, in one configuration of the system, the speed or the acceleration of the medical device can be controlled by controlling the speed at which the controlling line driver (not shown) releases the controlling line 2. The force exerted by the controlling line 2 can be determined from the speed/acceleration of the medical device.

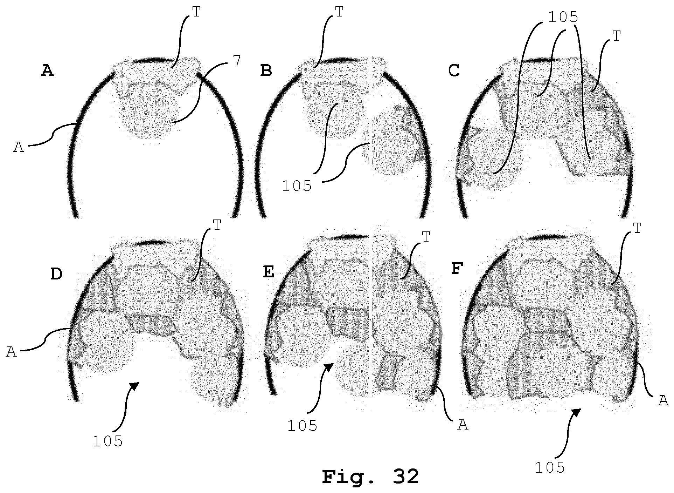

Fig. 32 schematically shows the treatment of an aneurysm A with thrombogenic filling elements 7. A device (not shown) was used to navigate, in this case autonomously, to the aneurysm A. The autonomous navigation is achieved by means of a navigation system which adjusts the forces applied on the device to follow the targeted trajectory. The balanced forces are in particular a magnetic force exerted by an external magnet, a drag force by blood in a vessel, and a gravity force.

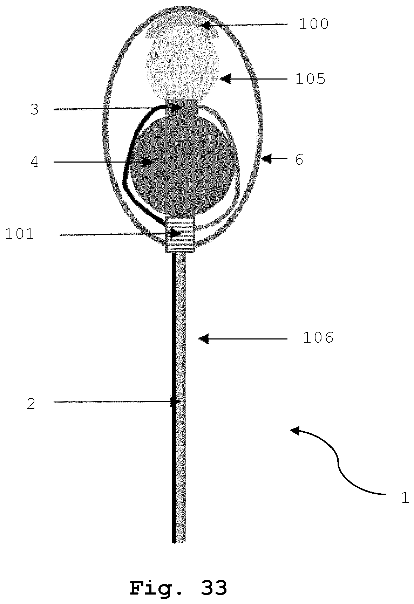

In panel A, a first filling element 105, corresponding to a distal part of a device (not shown, see Fig. 33a-33d), is shown inside an aneurysm A. The filling element 105 has both thrombogenic and expansive properties and is shown here after release from a proximal part of the device and expanded. A thrombus T has formed due to the thrombogenic properties of the filling element 105 and provides attachment of the same to the aneurysm wall. Panels B-F show, schematically, the progressive filling of the aneurysm A with more filling elements 105, which are identical here. It will be understood, however, that it may be advantageous in some circumstances to use different types of filling elements. Ultimately, as shown in panel F, the full volume of the aneurysm A is filled with filling elements 105 and thrombi T.

Fig. 33 schematically shows an embodiment of a device 1 according to another embodiment. The device 1 comprises a controlling line 2 with electrical wires 106 integrated therein. A magnetic part 4 is attached to the controlling line 2, forming a proximal part of the device 1. A combined thrombogenic and expansive element 105, forming a distal part, is attached to the proximal part by means of a release mechanism 3 which is in operable connection with the electrical wires 106 activatable by electrical wires. This avoids leaving the magnetic element 4 within the aneurysm A permanently as it is removable. The release mechanism 3 is biodegradable when a current is applied by the electrical wires 106. The filling element 105 is adapted to swell when hydrated and will thus self-expand when exposed to blood. The use of an expansive element allows to reduce the size of the filling element for navigation. A protective shell 6 is arranged around the magnetic part 4 and the filling element 105. Here, an activation mechanism 101 is shown which is usable to selectively open the protective shell 6 to avoid premature activation of the filling element functions. The activation element 101 here comprises a MEMS which is activatable by a current delivered by wires 106. The protective shell 6 is removable by breaking the shell 6 and pulling the shell 6 back by the activation element 101. Breaking of the shell 6 is achieved at a distal portion (generally in the vicinity of anchoring coating 100, see below) forming a breaking area. A thickness at the breaking area is lower than a general thickness of the shell 6. Here, the thickness of the breaking area is 70 $\mu$m and the thickness of the shell 6 is 200 $\mu$m. Alternatively, an electrical stimulus generated by an activation system embedded in the distal part of the device 1 may be used to remove the shell 6. Furthermore, an anchoring coating 100 is attached to a distal-most part of the filling element 105, but inside the protective shell 6. The anchoring coating provides the thrombogenicity in this example and may provide attachment to a vessel wall, thus ensuring that the filling element 105 does not get flushed out of the aneurysm A. The anchoring coating 100 here comprises collagen. Additionally or alternatively, the anchoring coating 100 may comprise or consist of thrombin, Von Wilerbrand factor (vWF), laminin, thrombospondin, vitronectin, fibrinogen, Thromboxane A2, or any combinations thereof. The thrombus T additionally fills the aneurysm and reduces the necessary volume of filling elements 105. The anchoring coating 100 has thrombin concentration of 600 NIH U/ml and thus induces the formation of a thrombus T with a fibrin content of approximately 60%, whichprovides advantageous mechanical properties of the thrombus such as reduced compaction.

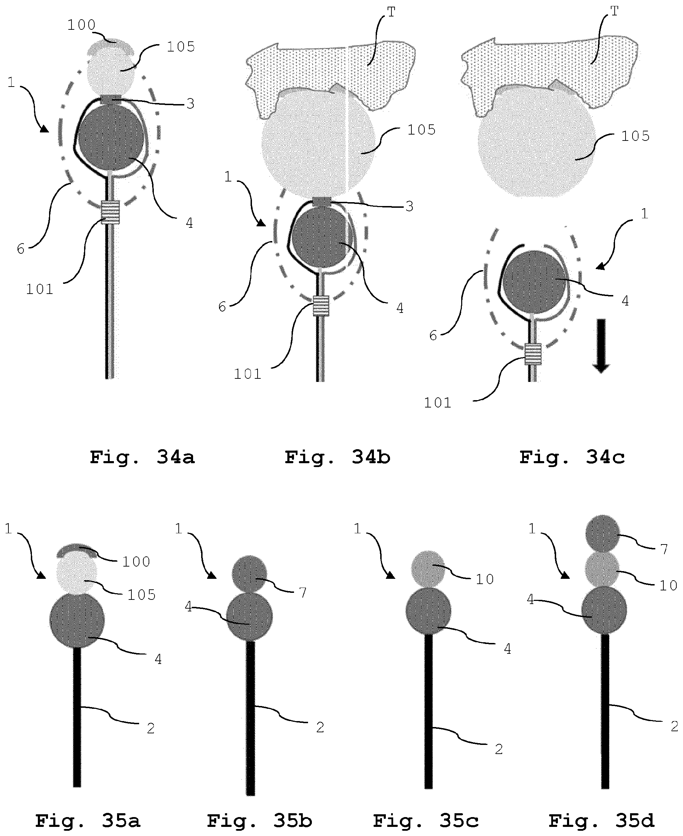

Fig. 34a shows the device 1 of Fig. 33 which may be used to deliver a filling element 105 into an aneurysm (not shown). The device 1 comprises a distal part made up of the filling element 105 and a proximal part comprising the magnetic part 4 and the controlling line 2. The filling element 105 is attached to the magnetic part 4 by means of the release mechanism 3 and has both expansive and thrombogenic properties, i.e. combines a thrombogenic element and an expansive element. The thrombogenic properties are provided by the anchoring coating 100. The protective shell 6 is arranged such as to surround the magnetic element 4 and the filling element 105. The anchoring coating 100 is arranged outside the protective shell 6 here because it has been partially or entirely activated such as to release the anchoring coating 100. As shown here, the device 1 may be delivered to an aneurysm. The protective shell 6 starts dissolving after being contacted with blood. Alternatively, the shell may be removed by any mechanism described herein, in particular by a mechanical or electrical stimulus.

Fig. 34b shows the device 1 in the position of Fig. 34a, wherein the filling element 105 has expanded due to exposure to blood. At the same time, a thrombus T has started to form and provides attachment to a vessel wall, allowing to release the release mechanism 3 by electrical current provides by cables in the controlling line 2.

Fig. 34c shows the retraction of the proximal part of the device 1 by a pulling force being exerted on the controlling line 2 after release of the release mechanism by biodegradation. The resulting configuration of the filling element 105, when inside the aneurysm (not shown), may substantially corresponds to panel A of Fig. 32 shown above.

Figs. 35a-35d show schematically different design options of a distal part of device 1. All devices 1 shown here have a controlling line 2 and a magnetic part 4 forming a proximal part, which will not be described separately again herein below for clarity. Further for clarity, the release mechanism is not shown, but may be any release mechanism disclosed herein.

Fig. 35a shows a device 1 with an expansive element 105 and an anchoring coating 100 forming a distal part of the device 1.

Fig. 35b shows an example of a device 1 where the distal part comprises a thrombogenic element 7.

Fig. 35c shows an example of a device 1 where the distal part comprises an expansive element 10.

Fig. 35d shows an alternative embodiment wherein the distal part of the device comprises an expansive element 10 attached to the magnetic part 4, and a thrombogenic element attached at a distal position of the expansive element 10. A release mechanism (not shown) may be arranged between the expansive element and the magnetic part 4 or between the controlling line 2 and the magnetic part 4.

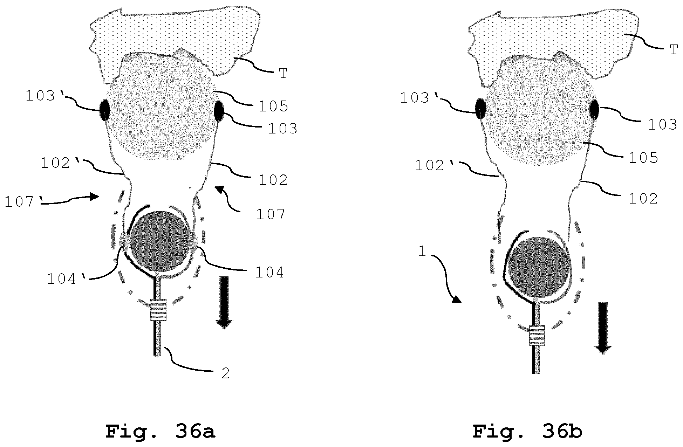

Fig. 36a shows an embodiment of a device 1 which is similar to the device 1 of Figs. 33 and 34a-34c. For clarity, identical features are not described again, but it will be understood that any function and feature described in this context above may also be present in the device of Fig. 36a. Here, the device 1 further comprises a second release mechanism 107, 107'. The second release mechanism comprises attachment points 104, 104' which are attached to the magnetic part 4 and connected to sutures 102, 102'. The sutures 102, 102' are attached to the filling element 105' by means of second attachment points 103, 103'. The sutures 102, 102' are adapted to hold the filling element 105 temporarily after release of the first release mechanism (not shown, see Fig. 33, ref. sign 3). Therefore, after the first detachment of the filling element 105, the proximal part of the device 1 is moved back by the controlling line 2. The position of the filling element 105 may be imaged to verify that it is indeed attached to a vessel wall.

Fig. 36b shows the final release of filling element 105, by means of dissolution of attachment points 104, 104' (not shown, see Fig. 36a). To this end, the attachment points comprise magnesium alloy which degrades when an electrical current is applied. Alternatively, iron-based alloys may be employed.

## Claims

1. A device (1) for treatment of a vessel (V) in a patient comprising

- a filling element (5,7,10,13,14,15,29)
- a magnetic element (4),
- a controlling line (2),
- a release mechanism (3),

wherein the release mechanism (3) is adapted to release the magnetic element (4) and the filling element (5,7,10,13,14,15,29) such as to be separated from the controlling line (2), wherein the magnetic element (4) is adapted to be guided by an external actuator (M) such as to guide at least the filling element (5,7,10,13,14,15,29) along a vessel path (V), wherein the filling element (5,7,10,13,14,15,29) is adapted to, when placed in the vessel (V) to be treated, reduce the volume of said vessel portion (V), wherein the filling element (5,7,10,13,14,15,29) is at least partially attached to, attachable to, or formed by the magnetic element (4) **characterised in that** the device is adapted to be carried by a flow of blood after insertion into a vessel, wherein the controlling line (2) is adapted to position the device by being loosened or pulled.

2. Device according to claim 1, wherein the filling element (5,7,10,13,14,15,29) is an expansive (10,13,15) and/or a thrombogenic element (5,7,14,29).

3. Device according to any claim 1 or 2, wherein the device (1) comprises an activation mechanism adapted to activate the filling element (5,7,10,13,14,15,29).

4. Device according to any one of the preceding claims, wherein the filling element (5,7,10,13,14,15,29) comprises at least one of a thrombogenic shape, a thrombogenic material, and a thrombogenic agent.

5. Device according to any one of the preceding claims, wherein the filling element (5,7,10,13,14,15,29) comprises at least one defined shape which is at least partially curved.

6. Device according to claim 5, wherein the filling element (5,7,10,13,14,15,29) comprise at least a first and second defined shape.

7. Device according to any one of the preceding claims, further comprising a configurable line (11) wherein the configurable line (11) forms a connection between the filling element (5,7,10,13,14,15,29) and the magnetic element (4).

8. Device according claim 7, wherein the configurable line (11) is adapted to be formed into a predefined configuration.

9. Device according to any one of the preceding claims, wherein the filling element (5,7,10,13,14,15,29) comprises a compressed foam (26) which is adapted to expand into an expanded shape, wherein the expanded shape substantially corresponds to the shape of an aneurysm (A).

10. Device according to any one of the preceding claims, further comprising a protective coating (6), in particular as part of an activation mechanism, preferably wherein the protective coating (6) is arranged radially outside of the filling element (5,7,10,13,14,15,29).

11. Device according to any one of claims 1-6, 9 or 10, wherein the filling element (5,7,10,13,14,15,29) is arranged radially on the outside of the magnetic element (4).

12. Device according to any one of claim 3, or claims 4-11 when depending on claim 3, wherein the release mechanism (3) is arranged in between the controlling line (2) and at least one or two or all of the filling element (5,7,10,13,14,15,29), magnetic element (4), and activation mechanism.

13. Device according to any one of the preceding claims, further comprising an anchoring element adapted to provide attachment between at least a part of the device with a blood vessel wall and/or a blood clot.

14. A system for treatment of a vessel (V) in a patient, preferably an aneurysm (A), in particular a neurovascular or aortic aneurysm (A), comprising a device according to any one of the preceding claims, further comprising at least one controlling line driver, a magnetic actuator (M), and a controlling unit.

15. The system according to claim 14, further wherein the magnetic actuator (M) is adapted to generate a, preferably predetermined, magnetic field at a preferably predetermined location in order to steer the device (1) in a preferably predetermined direction, wherein the controlling unit is adapted to balance, in particular in real-time, at least three forces applied on the medical device, preferably three forces including at least one of a flow drag force, a force from the controlling line (2), and a magnetic force by the magnetic actuator (M), and to operate the magnetic actuator (M) and/or the controlling line driver.

**Patentansprüche**

1. Vorrichtung (1) zur Behandlung eines Gefäßes (V) in einem Patienten, umfassend

- ein Füllelement (5,7,10,13,14,15,29)
- ein magnetisches Element (4),
- eine Kontrolllinie (2),
- ein Releasemechanismus (3),

wobei der Releasemechanismus (3) so ausgelegt ist, dass er das magnetische Element (4) und das Füllelement (5, 7, 10, 13, 14, 15, 29) so löst, dass sie von der Kontrollinie (2) getrennt werden, wobei das magnetische Element (4) so ausgelegt ist, dass es von einem externen Aktuator (M) geführt wird, um zumindest das Füllelement (5, 7, 10, 13, 14, 15, 29) entlang eines Gefäßpfades (V) zu führen, wobei das Füllelement (5, 7, 10, 13, 14, 15, 29) so ausgelegt ist, dass es, wenn es in das zu behandelnde Gefäß (V) eingebracht wird, das Volumen des Gefäßabschnitts (V) verringert, wobei das Füllelement (5, 7, 10, 13, 14, 15, 29) zumindest teilweise an dem magnetischen Element (4) befestigt, an diesem befestigbar oder durch dieses gebildet ist, **dadurch gekennzeichnet, dass** die Vorrichtung so ausgelegt ist, dass sie nach dem Einführen in ein Gefäß vom Blutstrom mitgeführt wird, wobei die Kontrolllinie (2) so ausgelegt ist, dass sie die Vorrichtung durch Lösen oder Ziehen positioniert.

2. Vorrichtung nach Anspruch 1, wobei das Füllelement (5, 7, 10, 13, 14, 15, 29) ein expansives (10, 13, 15) und/oder ein thrombogenes Element (5, 7, 14, 29) ist.

3. D Vorrichtung nach einem der Ansprüche 1 oder 2, wobei die Vorrichtung (1) einen Aktivierungsmechanismus umfasst, der dazu ausgelegt ist, das Füllelement (5, 7, 10, 13, 14, 15, 29) zu aktivieren.

4. Vorrichtung nach einem der vorstehenden Ansprüche, wobei das Füllelement (5, 7, 10, 13, 14, 15, 29) mindestens eines von einer thrombogenen Form, einem thrombogenen Material und einem thrombogenen Wirkstoff umfasst.

5. Vorrichtung nach einem der vorstehenden Ansprüche, wobei das Füllelement (5, 7, 10, 13, 14, 15, 29) mindestens eine definierte Form aufweist, die zumindest teilweise gekrümmt ist.

6. Vorrichtung nach Anspruch 5, wobei das Füllelement (5, 7, 10, 13, 14, 15, 29) mindestens eine erste und eine zweite definierte Form aufweist.

7. Vorrichtung nach einem der vorstehenden Ansprüche, die ferner eine konfigurierbare Leitung (11) umfasst, wobei die konfigurierbare Leitung (11) eine Verbindung zwischen dem Füllelement (5, 7, 10, 13, 14, 15, 29) und dem magnetischen Element (4) bildet.

8. Vorrichtung nach Anspruch 7, wobei die konfigurierbare Leitung (11) so ausgelegt ist, dass sie zu einer vordefinierten Konfiguration geformt werden kann.

9. Vorrichtung nach einem der vorstehenden Ansprüche, wobei das Füllelement (5, 7, 10, 13, 14, 15, 29) einen komprimierten Schaumstoff (26) umfasst, der so ausgelegt ist, dass er sich zu einer expandierten Form ausdehnt, wobei die expandierte Form im Wesentlichen der Form eines Aneurysmas (A) entspricht.

10. Vorrichtung nach einem der vorstehenden Ansprüche, die ferner eine Schutzbeschichtung (6) umfasst, insbesondere als Teil eines Aktivierungsmechanismus, wobei die Schutzbeschichtung (6) vorzugsweise radial außerhalb des Füllelements (5, 7, 10, 13, 14, 15, 29) angeordnet ist.

11. Vorrichtung nach einem der Ansprüche 1 bis 6, 9 oder 10, wobei das Füllelement (5, 7, 10, 13, 14, 15, 29) radial an der Außenseite des Magnetelements (4) angeordnet ist.

12. Vorrichtung nach einem der Ansprüche 3 oder, in Abhängigkeit von Anspruch 3, nach den Ansprüchen 4 bis 11, wobei der Auslösemechanismus (3) zwischen der Kontrolllinie (2) und mindestens einem oder zwei oder allen der Füllelemente (5, 7, 10, 13, 14, 15, 29), dem Magnetelement (4) und dem Aktivierungsmechanismus angeordnet ist.

13. Vorrichtung nach einem der vorstehenden Ansprüche, die ferner ein Verankerungselement umfasst, das dazu ausgelegt ist, eine Befestigung zwischen mindestens einem Teil der Vorrichtung und einer Blutgefäßwand und/oder einem Blutgerinnsel zu bewirken.

14. System zur Behandlung eines Gefäßes (V) in einem Patienten, vorzugsweise eines Aneurysmas (A), insbesondere eines neurovaskulären oder aortalen Aneurysmas (A), umfassend eine Vorrichtung gemäß einem der vorstehenden Ansprüche, ferner umfassend mindestens einen Kontrolllinientreiber, einen magnetischen Aktuator (M) und eine Steuereinheit.

**15.** Das System gemäß Anspruch 14, wobei der Magnetaktuator (M) ferner dazu ausgelegt ist, ein vorzugsweise vorbestimmtes Magnetfeld an einem vorzugsweise vorbestimmten Ort zu erzeugen, um die Vorrichtung (1) in eine vorzugsweise vorbestimmte Richtung zu lenken, wobei die Steuereinheit dazu ausgelegt ist, insbesondere in Echtzeit mindestens drei auf die medizinische Vorrichtung ausgeübte Kräfte, vorzugsweise drei Kräfte, die mindestens eine Strömungswiderstandskraft, eine Kraft von der Kontrolllinie (2) und eine Magnetkraft durch den Magnetaktuator (M) umfassen, auszugleichen und den Magnetaktuator (M) und/oder den Kontrolllinientreiber zu betreiben.

**Revendications**

**1.** Dispositif (1) pour le traitement d'un vaisseau (V) chez un patient comprenant

- un élément de remplissage (5,7,10,13,14,15,29)
- un élément magnétique (4),
- une ligne de contrôle (2),
- un mécanisme de libération (3),

dans lequel le mécanisme de libération (3) est adapté pour libérer l'élément magnétique (4) et l'élément de remplissage (5, 7, 10, 13, 14, 15, 29) de manière à être séparés de la ligne de contrôle (2), dans lequel l'élément magnétique (4) est adapté pour être guidé par un actionneur externe (M) de manière à guider au moins l'élément de remplissage (5, 7, 10, 13, 14, 15, 29) le long d'un trajet de vaisseau (V), dans lequel l'élément de remplissage (5, 7, 10, 13, 14, 15, 29) est adapté pour, lorsqu'il est placé dans le vaisseau (V) à traiter, réduire le volume de ladite partie du vaisseau (V), dans lequel l'élément de remplissage (5, 7, 10, 13, 14, 15, 29) est au moins partiellement fixé à, peut être fixé à, ou est formé par l'élément magnétique (4) **caractérisé en ce que** le dispositif est adapté pour être transporté par un flux sanguin après insertion dans un vaisseau, dans lequel la ligne de commande (2) est adaptée pour positionner le dispositif en étant détendue ou tirée.

**2.** Dispositif selon la revendication 1, dans lequel l'élément de remplissage (5, 7, 10, 13, 14, 15, 29) est un élément expansible (10, 13, 15) et/ou un élément thrombogène (5, 7, 14, 29).

**3.** Dispositif selon l'une quelconque des revendications 1 ou 2, dans lequel le dispositif (1) comprend un mécanisme d'activation adapté pour activer l'élément de remplissage (5, 7, 10, 13, 14, 15, 29).

**4.** Dispositif selon l'une quelconque des revendications précédentes, dans lequel l'élément de remplissage (5, 7, 10, 13, 14, 15, 29) comprend au moins l'un parmi une forme thrombogène, un matériau thrombogène et un agent thrombogène.

**5.** Dispositif selon l'une quelconque des revendications précédentes, dans lequel l'élément de remplissage (5, 7, 10, 13, 14, 15, 29) comprend au moins une forme définie qui est au moins partiellement courbée.

**6.** Dispositif selon la revendication 5, dans lequel l'élément de remplissage (5, 7, 10, 13, 14, 15, 29) comprend au moins une première et une deuxième formes définies.

**7.** Dispositif selon l'une quelconque des revendications précédentes, comprenant en outre une ligne configurable (11), dans lequel la ligne configurable (11) forme une connexion entre l'élément de remplissage (5, 7, 10, 13, 14, 15, 29) et l'élément magnétique (4).

**8.** Dispositif selon la revendication 7, dans lequel la ligne configurable (11) est adaptée pour être formée en une configuration prédéfinie.

**9.** Dispositif selon l'une quelconque des revendications précédentes, dans lequel l'élément de remplissage (5, 7, 10, 13, 14, 15, 29) comprend une mousse comprimée (26) qui est adaptée pour se dilater en une forme expansée, dans lequel la forme expansée correspond sensiblement à la forme d'un anévrisme (A).

**10.** Dispositif selon l'une quelconque des revendications précédentes, comprenant en outre un revêtement protecteur (6), en particulier dans le cadre d'un mécanisme d'activation, de préférence dans lequel le revêtement protecteur (6) est disposé radialement à l'extérieur de l'élément de remplissage (5, 7, 10, 13, 14, 15, 29).

11. Dispositif selon l'une quelconque des revendications 1 à 6, 9 ou 10, dans lequel l'élément de remplissage (5, 7, 10, 13, 14, 15, 29) est disposé radialement à l'extérieur de l'élément magnétique (4).

12. Dispositif selon l'une quelconque des revendications 3 ou 4 à 11, en dépendance de la revendication 3, dans lequel le mécanisme de libération (3) est disposé entre la ligne de commande (2) et au moins un ou deux ou tous les éléments de remplissage (5, 7, 10, 13, 14, 15, 29), l'élément magnétique (4) et le mécanisme d'activation.

13. Dispositif selon l'une quelconque des revendications précédentes, comprenant en outre un élément d'ancrage adapté pour assurer la fixation entre au moins une partie du dispositif et une paroi vasculaire et/ou un caillot sanguin.

14. Système pour le traitement d'un vaisseau (V) chez un patient, de préférence un anévrisme (A), en particulier un anévrisme neurovasculaire ou aortique (A), comprenant un dispositif selon l'une quelconque des revendications précédentes, comprenant en outre au moins un pilote de ligne de commande, un actionneur magnétique (M) et une unité de commande.

15. Le système selon la revendication 14, dans lequel l'actionneur magnétique (M) est en outre adapté pour générer un champ magnétique, de préférence prédéterminé, à un emplacement de préférence prédéterminé afin de diriger le dispositif (1) dans une direction de préférence prédéterminée, dans lequel l'unité de commande est adaptée pour équilibrer, en particulier en temps réel, au moins trois forces appliquées sur le dispositif médical, de préférence trois forces comprenant au moins l'une parmi une force de traînée d'écoulement, une force provenant de la ligne de commande (2), et une force magnétique exercée par l'actionneur magnétique (M), et à actionner l'actionneur magnétique (M) et/ou le circuit d'attaque de la ligne de commande.

Fig. 1a

Fig. 1b

Fig. 1c

Fig. 1d

Fig. 1e

Fig. 1f

Fig. 1g

Fig. 1h

Fig. 1i

Fig. 2a        Fig. 2b        Fig. 2c        Fig. 2d

Fig. 3a     Fig. 3b     Fig. 3c     Fig. 3d     Fig. 3e

Fig. 4a     Fig. 4b     Fig. 4c     Fig. 4d     Fig. 4e

Fig. 5a

Fig. 5b

Fig. 6a

Fig. 6b

Fig. 6c

Fig. 7a      Fig.7b      Fig. 7c      Fig. 7d

Fig. 8a     Fig. 8b     Fig. 8c     Fig. 8d     Fig. 8e

Fig. 9a     Fig. 9b     Fig. 9c     Fig. 9d     Fig. 9e

Fig. 9f

Fig. 10a      Fig. 10b      Fig. 10c      Fig. 10d

Fig. 10e      Fig. 10f      Fig. 10g      Fig. 10h

Fig. 11a                    Fig. 11b

Fig. 12

Fig. 13a          Fig. 13b          Fig. 13c

Fig. 14

Fig. 15

Fig. 16

Fig. 17

Fig. 18

Fig. 19

Fig. 20a

Fig. 20b

Fig. 21

Fig. 22

Fig. 23a      Fig. 23b      Fig. 23c      Fig. 23d

Fig. 24  Fig. 25a  Fig. 25b

Fig. 26a  Fig. 26b

Fig. 27

Fig. 28a    Fig. 28b

Fig. 29

Fig. 30

Fig. 31

Fig. 32

Fig. 33

**Fig. 34a**          **Fig. 34b**          **Fig. 34c**

**Fig. 35a**       **Fig. 35b**       **Fig. 35c**       **Fig. 35d**

Fig. 36a

Fig. 36b

**REFERENCES CITED IN THE DESCRIPTION**

**Patent documents cited in the description**

- US 6315709 B
- US 6530934 B
- US 2002087077 A1
- WO 0054832 A1